# EUROPEAN PATENT SPECIFICATION

(11) **EP 2 494 069 B1**
(45) Date of publication and mention of the grant of the patent: **02.10.2013**
(21) Application number: 10773584.7
(22) Date of filing: 29.10.2010
(51) Int. Cl.: C12Q 1/68

(54) **METHOD FOR DETECTING BALANCED CHROMOSOMAL ABERRATIONS IN A GENOME**
VERFAHREN ZUM NACHWEIS VON AUSGEGLICHENEN CHROMOSOMALEN ABERRATIONEN IN GENOMEN
PROCÉDÉ DE DÉTECTION DES ABERRATIONS CHROMOSOMIQUES ÉQUILIBRÉES

(30) Priority: 30.10.2009 EP 09013670
(43) Date of publication of application: 05.09.2012
(73) Proprietor: Roche Diagnostics GmbH, 68305 Mannheim (DE); F.Hoffmann-La Roche AG, 4070 Basel (CH)
(72) Inventor: DUGAS, Martin, 48163 Münster (DE); GROSSMANN, Vera, 81375 München (DE); HAFERLACH, Claudia, 82266 Inning (DE); HAFERLACH, Torsten, 82266 Inning (DE); KERN, Wolfgang, 82319 Starnberg (DE); KLEIN, Hans-Ulrich, 48145 Münster (DE); KOHLMANN, Alexander, 92318 Neumarkt i.d. OPf (DE); SCHNITTGER, Susanne, 81377 München (DE)
(74) Representative: Bösl, Raphael Konrad
(86) International application number: PCT/EP2010/006627
(87) International publication number: WO 2011/050981

(56) References cited:
- WO-A1-99/23251
- WO-A1-2006/113613
- WO-A1-2009/053039
- WO-A1-2009/091879
- WO-A2-03/044486
- US-A- 5 487 970
- KEHRER-SAWATZKI H ET AL: "THE SECOND CASE OF A T(17;22) IN A FAMILY WITH NEUROFIBROMATOSIS TYPE 1: SEQUENCE ANALYSIS OF THE BREAKPOINT REGIONS", HUMAN GENETICS, SPRINGER, BERLIN, DE, vol. 99, no. 2, 1 February 1997 (1997-02-01), pages 237-247, XP002071723, ISSN: 0340-6717

## Description

### Field of the Invention

The present application relates to the field of enrichment and analysis of nucleic acid sequences by means of capturing said sequences. More precisely, the present invention provides a new method to capture specific genomic regions for subsequent sequence analysis in order to detect balanced chromosomal aberrations, especially if the region of interest is too large to be amplified by only one or a few polymerase chain reactions (PCR). In particular, this new method is suitable to identify fusion partner genes, occurring in cancer genomes. Moreover, this novel method allows identifying thus far unknown fusion partner genes, i.e. by capturing one fusion partner but also sequencing the second fusion partner as a result of such a chimeric sequence. This invention can, in particular, be applied both for translocations and inversions.

### Background of the Invention

The advent of nucleic acid microarray technology, for example DNA microarray technology, makes it possible to build an array of millions of nucleic acid sequences, for example DNA sequences, in a very small area, for example on a microscope slide, as e.g. disclosed in US Patent Nos. 6,375,903 and 5,143,854. Initially, such arrays were created by spotting pre-synthesized DNA sequences onto slides. However, the construction of maskless array synthesizers (MAS) in which light is used to direct synthesis of the DNA sequences, the light direction being performed using a digital micromirror device (DMD), as described in US Patent No. 6,375,903, now allows for the *in situ* synthesis of oligonucleotide sequences directly on the slide itself.

Using a MAS instrument, the selection of oligonucleotide sequences or DNA sequences to be constructed on the microarray is under software control such that it is now possible to create individually customized arrays based on the particular needs of an investigator. In general, MAS-based oligonucleotide or DNA microarray synthesis technology allows for the parallel synthesis of millions of unique oligonucleotide features in a very small area of a standard microscope slide. The microarrays are generally synthesized by using light to direct which oligonucleotides are synthesized at specific locations on an array, these locations being called features.

With the availability of the entire genomes of hundreds of organisms, for which a reference sequence has generally been deposited into a public database, microarrays have been used to perform sequence analysis on nucleic acids or DNA isolated from a myriad of organisms.

Nucleic acid or DNA microarray technology has been applied to many areas of basic research and clinical diagnostics, such as gene expression profiling and biomarker discovery (Haferlach T et al. Blood. 2005 Aug 15;106(4):1189-98.), mutation detection, allelic and evolutionary sequence comparison, genome mapping, drug discovery, and more. Many applications require searching for genetic aberrations and point mutations across the entire human genome that underlie human diseases. In the case of complex diseases, these searches generally result in a single nucleotide polymorphism (SNP) or set of SNPs associated with diseases and/or disease risk. Identifying such SNPs has proved to be an arduous and frequently fruitless task because resequencing large regions of genomic DNA, usually greater than 100 kilobases (Kb), from affected individuals or tissue samples is required to find a single base change or to identify all sequence variants. Resequencing genomic DNA also is required to characterize patient samples with respect to small insertions and deletions. These micro-aberrations have been proven to be of diagnostic and prognostic relevance (Schnittger S et al., Blood. 2002 Jul 1;100(1):59-66).

Other applications involve the identification of gains and losses of chromosomal sequences which may also be associated with cancer, such as leukemia (Walter MJ et al., Proc Natl Acad Sci U S A. 2009 Aug 4;106(31):12950-5.) or lymphoma (Martinez-Climent JA et al., 2003, Blood 101:3109-3117), gastric cancer (Weiss MM et al., 2004, Cell. Oncol. 26:307-317), breast cancer (Callagy G et al., 2005, J. Path. 205: 388-396) and prostate cancer (Paris, PL et al., 2004, Hum. Mol. Gen. 13:1303-1313). As such, microarray technology is a tremendously useful tool for scientific investigators and clinicians in their understanding of diseases and therapeutic regimen efficacy in treating diseases.

The genome is typically too complex to be studied as a whole, and techniques must be used to reduce the complexity of the genome. To address this problem, one solution is to reduce certain types of abundant sequences from a genomic nucleic acid or DNA sample, as found in US Patent 6,013,440. Alternatives employ methods and compositions for enriching genomic sequences as described, for example, in Albert et al. (2007, Nat. Meth., 4:903-5), Okou et al. (2007, Nat. Meth. 4:907-9), Olson M. (2007, Nat. Meth. 4:891-892), Hodges et al. (2007, Nat. Genet. 39:1522-1527) and as found in United States Patent Applications US 2007/0196843, US 2008/0194414, and US 2009/0221438. Albert et al. disclose an alternative that is both cost-effective and rapid in effectively reducing the complexity of a genomic sample in a user defined way to allow for further processing and analysis. Lovett et al. (1991, Proc. Natl. Acad. Sci. 88:9628-9632) also describes a method for genomic selection using a bacterial artificial chromosomes.

Microarray technology, be it enrichment technology or otherwise, is typically a substrate associated technology with inherent variability, such as microarray slides, chips, and the like. Variability can take on many forms, for example variability in background, probe/hybridization kinetics, glass source, and the like.

WO 2009/053039 discloses a general method for capturing and enrichment of target nucleic acids for reducing the complexity of target nucleic acids, preferably a genomic sample, for further analysis such as direct DNA sequencing, resequencing or SNP calling.

In cancer genomes various chromosomal aberrations are found (see for example Atlas of Genetics and Cytogenetics in Oncology and Haematology. URL http://AtlasGeneticsOncology.org). In particular in leukemia, balanced chromosomal aberrations like translocations and inversions have been proven to have diagnostic and prognostic relevance. For example, a subset of human acute leukemias with a decidedly unfavorable prognosis possesses a chromosomal translocation involving the "Mixed Lineage Leukemia" *(MLL, HRX, AU-1)* gene on chromosome segment 11q23. The leukemic cells have been classified as "Acute Lymphoblastic Leukemia" (ALL). However, unlike the majority of childhood ALL, the presence of the *MLL* translocations often results in an early relapse after chemotherapy. Generally, therapeutic treatment is more successful when tailored to the specific type of cancer, in particular with respect to leukemia. Today, the genetic characterization necessary for optimal treatment of acute myeloid leukemia (AML) requires a combination of different labor-intensive methods such as chromosome banding analysis, sequencing for the detection of molecular mutations, and RT-PCR for the confirmation of characteristic fusion genes. Thus, a need exists for accurate and efficient methods for diagnosis of malignancies like leukemia, and for identifying their subclasses other than conventional assays such as metaphase cytogenetics or fluorescence in situ hybridization (FISH).

### Summary of the Invention

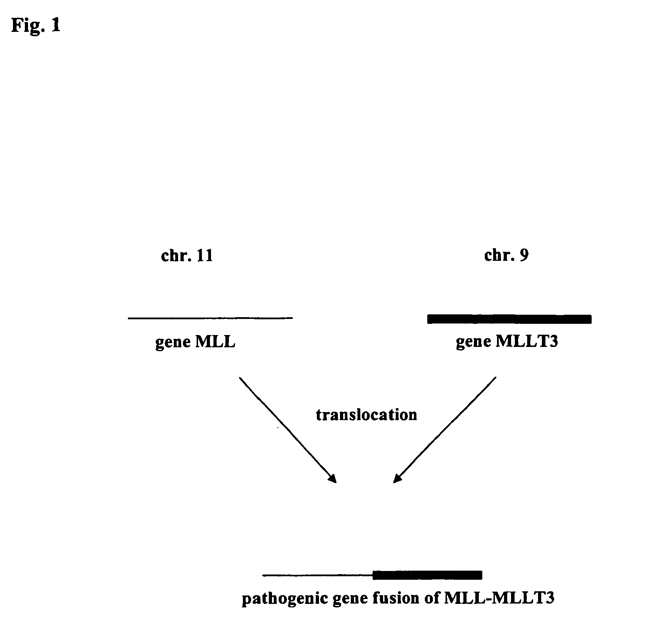

The present invention provides methods and systems for the capture and enrichment of target nucleic acids and analysis of the enriched target nucleic acids for detecting balanced chromosomal aberrations including translocations and inversions (see Fig. 1). In particular, the present invention provides for the enrichment of targeted sequences in a format by representing one fusion partner gene on a capturing platform and to allow subsequent sequencing of chimeric nucleic acids, i.e. nucleic acid strands that carry information on different DNA regions of a genome. Surprisingly, such a design allowed the identification of novel fusion partner genes occurring as a result of a chromosomal translocation or inversion.

Therefore, one embodiment of the present invention as defined in the appended claims is directed to a method for detecting balanced chromosomal aberrations in a genome, the method comprising the steps of:
(a) exposing fragmented, denatured nucleic acid molecules of said genome to multiple, different oligonucleotide probes located on multiple, different sites of a solid support under hybridizing conditions to capture nucleic acid molecules that specifically hybridize to said probes,
   wherein said fragmented, denatured nucleic acid molecules have an average size of about 100 to about 1000 nucleotide residues, preferably about 250 to about 800 nucleotide residues and most preferably about 400 to about 600 nucleotide residues, in particular about 500 nucleotide residues,
   wherein said oligonucleotide probes have an average size of about 20 to about 100 nucleotides, preferably about 40 to about 85 nucleotides, more preferred about 45 to about 75 nucleotides, in particular about 55 to about 65 nucleotide residues or about 60 nucleotide residues,
(b) separating unbound and non-specifically hybridized nucleic acids from the captured molecules;
(c) eluting the captured molecules from the solid support,
(d) optionally repeating steps (a) to (c) for at least one further cycle with the eluted captured molecules,
(e) determining the nucleic acid sequence of the captured molecules, in particular by means of performing sequencing by synthesis reactions,
(f) comparing the determined sequence to sequences in a database of the reference genome,
(g) identifying sequences in the determined sequence which only partially match or do not match with sequences of the reference genome,
(h) detecting at least one balanced chromosomal aberration.

Specific Embodiments of the present invention comprise (pre-selected) immobilized nucleic acid probes to capture target nucleic acid sequences from, for example, a genomic sample by hybridizing the sample to probes on a solid support. The captured target nucleic acids are preferably washed and eluted off of the probes. The eluted genomic sequences are more amenable to detailed genetic analysis than a sample that has not been subjected to the methods described herein.

An alternative embodiment of the present invention is directed to the solution based capture method comprising probe derived amplicons wherein said probes for amplification are affixed to a solid support. The solid support comprises support-immobilized nucleic acid probes to capture specific nucleic acid sequences from a genomic sample. Probe amplification provides probe amplicons in solution which are hybridized to target sequences. Following hybridization of probe amplicons to target sequences, target nucleic acid sequences present in the sample are enriched by capturing and washing the probes and eluting the hybridized target nucleic acids from the captured probes. The target nucleic acid sequence(s) may be further amplified using, for example, non-specific ligation-mediated PCR (LM-PCR), resulting in an amplified pool of PCR products of reduced complexity compared to the original target sample which is further analysed by sequencing as described above.

Consequently, the present invention broadly relates to cost-effective, flexible and rapid methods for reducing nucleic acid sample complexity to enrich for target nucleic acids of interest and to facilitate further processing and the identification of fusion or chimeric genes. Generally, the present invention provides methods useful, for example, in searching for genetic variants and mutations, single nucleotide polymorphisms (SNPs), sets of SNPs, genomic insertions and deletions in addition to the identification of balanced chromosomal aberrations.

### Definitions

As used herein, the term "about" refers to a general error range of +/- 10%, in particular +/-5%.

As used herein, the term "balanced chromosomal aberration" refers to chromosomal aberrations without visible gain or loss of genetic material, i.e. it refers to the rearrangement of genes, genomes or chromosomes without visible gain or loss of genetic material, whereas the term "unbalanced chromosomal aberrations" refers to aberrations with visible gain or loss of genetic material, i.e. aberrations with partial deletions or with loss of whole chromosomes. Examples of primary balanced chromosomal aberrations are translocations, in particular reciprocal translocations, and inversions. Balanced chromosomal aberrations usually lead to the formation of abnormal chimeric genes, i.e. genes containing at least two different chromosomal sections. According to the present invention, the term "balanced chromosomal aberration" include genetic rearrangements with partial deletions at the breaking points. The term "visible gain or loss of genetic material" in this context means the detection of gain or loss of genetic material by means of visible methods like metaphase cytogenetics or *in situ* hybridization of interface nuclei, e.g. fluorescence or chromatic *in situ* hybridization.

A used herein, the terms "chimeric gene" and "fusion gene" are used interchangeably and refer to a balanced chromosomal aberration of a gene or genomic region as explained above.

As used herein, the term "gene" or "gene of an organism" means the genomic or chromosomal DNA sequence containing at least one gene.

As used herein, the term "genetic material", "genetic sequence", "genomic material" or "genomic sequence" are used interchangeably and refer to chromosomal DNA.

As used herein, the term "hybridization" is used in reference to the pairing of complementary nucleic acids. Hybridization and the strength of hybridization (e.g., the strength of the association between the nucleic acids) is affected by such factors as the degree of complementary between the nucleic acids, stringency of the conditions involved, the melting temperature (Tₘ) of the formed hybrid, and the G:C ratio of the nucleic acids. While the present invention is not limited to a particular set of hybridization conditions, stringent hybridization conditions are preferably employed. Stringent hybridization conditions are sequence dependent and differ with varying environmental parameters (e.g., salt concentrations, presence of organics, etc.). Generally, "stringent" conditions are selected to be about 50°C to about 20°C lower than the Tₘ for the specific nucleic acid sequence at a defined ionic strength and pH. Preferably, stringent conditions are about 5°C to 10°C lower than the thermal melting point for a specific nucleic acid bound to a complementary nucleic acid. The Tₘ is the temperature (under defined ionic strength and pH) at which 50% of a nucleic acid (e.g., target nucleic acid) hybridizes to a perfectly matched probe.

As used herein, the term "isolate" when used in relation to a nucleic acid, as in "isolating a nucleic acid" refers to a nucleic acid sequence that is identified and separated from at least one component or contaminant with which it is ordinarily associated in its natural source. The isolated nucleic acid, oligonucleotide, or polynucleotide may be present in single-stranded or double-stranded form.

As used herein, the term "primer" refers to an oligonucleotide, whether occurring naturally as in a purified restriction digest or produced synthetically, that is capable of acting as a point of initiation of synthesis when placed under conditions in which synthesis of a primer extension product that is complementary to a nucleic acid strand is induced, (e.g., in the presence of nucleotides and an inducing agent such as DNA polymerase and at a suitable temperature and pH). The primer is preferably single stranded for maximum efficiency in amplification. Preferably, the primer is an oligodeoxyribonucleotide. The primer must be sufficiently long to prime the synthesis of extension products in the presence of the inducing agent. The exact lengths of the primers will depend on many factors, including temperature, source of primer and the use of the method.

As used herein, the term "probe" refers to an oligonucleotide whether occurring naturally as in a purified restriction digest or produced synthetically, recombinantly or by PCR amplification, that is capable of hybridizing to at least a portion of another oligonucleotide of interest, for example target nucleic acid sequences. A probe is generally single-stranded. Probes are useful in the detection, identification and isolation of particular gene sequences.

As used herein, the term "sample" is used in its broadest sense. In one sense, it is meant to include a specimen or culture obtained from any source, preferentially a biological source. Biological samples may be obtained from animals (including humans) and encompass fluids, solids, tissues, and gases. Biological samples include blood products, such as plasma, serum and the like. As such, a "sample of nucleic acids" or a "nucleic acid sample", a "target sample" comprises nucleic acids (e.g., DNA, RNA, cDNA, mRNA, tRNA, miRNA, etc.) from any source. According to the present invention, a nucleic acid sample preferably derives from a biological source, such as a human or non-human cell, tissue, and the like. The term "non-human" refers to all non-human animals and entities including, but are not limited to, vertebrates such as rodents, non-human primates, ovines, bovines, ruminants, lagomorphs, porcines, caprines, equines, canines, felines, aves, etc. Non-human also includes invertebrates and prokaryotic organisms such as bacteria, plants, yeast, viruses, and the like. As such, a nucleic acid sample used in methods and systems of the present invention is a nucleic acid sample derived from any organism, either eukaryotic or prokaryotic. "Stringent conditions" or "high stringency conditions," for example, can be hybridization in 50% formamide, 5x SSC (0.75 M NaCl, 0.075 M sodium citrate), 50 mM sodium phosphate (pH 6.8), 0.1 % sodium pyrophosphate, 5x Denhardt's solution, sonicated salmon sperm DNA (50 mg/ml), 0.1 % SDS, and 10% dextran sulfate at 42°C, with washes at 42°C in 0.2 % SSC (sodium chloride/sodium citrate) and 50% formamide at 55°C, followed by a wash with 0.1x SSC containing EDTA at 55°C. By way of example, but not limitation, it is contemplated that buffers containing 35% formamide, 5x SSC, and 0.1% (w/v) sodium dodecyl sulfate (SDS) are suitable for hybridizing under moderately non-stringent conditions at 45°C for 16-72 hours. Furthermore, it is envisioned that the formamide concentration may be suitably adjusted between a range of 20-45% depending on the probe length and the level of stringency desired. Additional examples of hybridization conditions are provided in several laboratory manual known for a person skilled in the art. Similarly, "stringent" wash conditions are ordinarily determined empirically for hybridization of a target to a probe, or a probe derived amplicon. The amplicon/target are hybridized (for example, under stringent hybridization conditions) and then washed with buffers containing successively lower concentrations of salts, or higher concentrations of detergents, or at increasing temperatures until the signal-to-noise ratio for specific to non-specific hybridization is high enough to facilitate detection of specific hybridization. Stringent temperature conditions will usually include temperatures in excess of about 30°C, more usually in excess of about 37°C, and occasionally in excess of about 45°C. Stringent salt conditions will ordinarily be less than about 1000 mM, usually less than about 500 mM, more usually less than about 150 mM (Wetmur et al., 1966, J. Mol. Biol., 31:349-370; Wetmur, 1991, Critical Reviews in Biochemistry and Molecular Biology, 26:227-259).

As used herein, the term "target nucleic acid molecules" and "target nucleic acid sequences" are used interchangeably and refer to molecules or sequences from a target genomic region to be studied. The pre-selected probes determine the range of targeted nucleic acid molecules. Thus, the "target" is sought to be sorted out from other nucleic acid sequences. A "segment" is defined as a region of nucleic acid within the target sequence, as is a "fragment" or a "portion" of a nucleic acid sequence.

### Detailed Description of the Invention

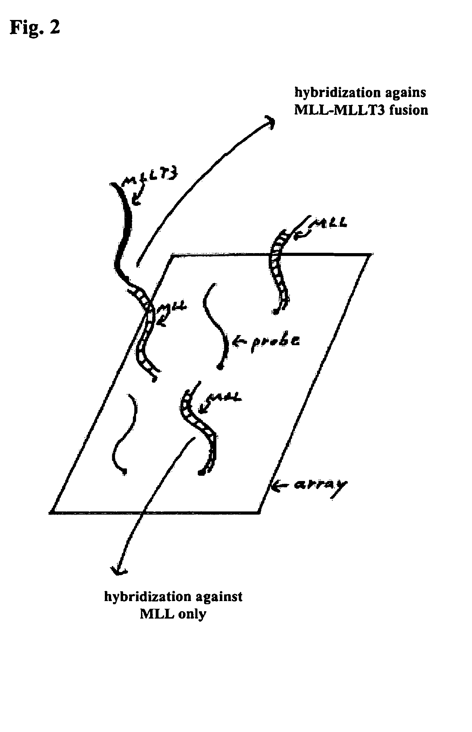

As outlined above, the first step (a) in the method of the present invention concerns exposing fragmented, denatured nucleic acid molecules of said genome to multiple, different oligonucleotide probes located on multiple, different sites of a solid support under hybridizing conditions to capture nucleic acid molecules that specifically hybridize to said probes (see Fig. 2).

The fragmented, denatured nucleic acid molecules have an average size of about 100 to about 1000 nucleotide residues, preferably about 250 to about 800 nucleotide residues and most preferably about 400 to about 600 nucleotide residues, in particular about 500 nucleotide residues. The nucleic acid fragments of random- or non-random size can generally be produced by methods known to a person skilled in the art, e.g. by chemical, physical or enzymatic fragmentation. Chemical fragmentation can employ metal ions and complexes (e.g., Fe-EDTA). Physical methods can include sonication, hydrodynamic shearing or nebulization (see e.g. European patent application EP 0 552 290). Enzymatic protocols can employ nucleases such as micrococcal nuclease (Mnase) or exo-nucleases (such as ExoI or Bal31) or restriction endonucleases. According to a preferred embodiment, the genomic DNA is fragmented by mechanical stress, in particular by sonication. The desired average size of the DNA fragments shall be small (≤ 1000 bp, in particular about 500 bp) and depends on the sequencing method to be applied.

In general, the nucleic acid molecules to be fragmented are genomic DNA molecules, in particular containing the whole genome or at least one gene or chromosome of an organism, or at least one genomic nucleic acid molecule with a size of at least about 50 kb, at least about 200 kb, at least about 500 kb, at least about 1 Mb, at least about 2 Mb or at least about 5 Mb, especially a size between about 100 kb and about 5 Mb, between about 200 kb and about 5 Mb, between about 500 kb and about 5 Mb, between about 1 Mb and about 2 Mb or between 2 Mb and about 5 Mb.

Denaturation of the nucleic acid fragments to a single-stranded state can be carried out using, for example, a chemical or thermal denaturing process.

The oligonucleotide probes have an average size of about 20 to about 100 nucleotides, preferably about 40 to about 85 nucleotides, more preferred about 45 to about 75 nucleotides, in particular about 55 to about 65 nucleotide residues or about 60 nucleotide residues. The probes usually define a plurality of exons, introns or regulatory sequences from a plurality of genetic loci because fusion breakpoints can occur both in introns and exons of genes. Therefore, probes are designed to cover as much as possible contiguous region of a gene of interest. Such multiple probes should preferably define the complete sequence of at least one single genetic locus of an organism, said locus having a size of at least 50 kb, in particular of at least 100 kb, preferably at least 1 Mb or at least one of the sizes as specified in claim 8, or from at least one gene or at least one chromosome of an organism, preferably from at least about 90%, in particular at least about 95%, especially at least about 98% of the gene or genome of an organism, in particular a human gene or genome. Said multiple probes can define sites known to contain a balanced chromosomal aberration. The multiple probes can also define a tiling array. Such a tiling array in the context of the present invention is defined as being designed to capture the complete sequence of at least one complete chromosome. In this context, the term "define" is understood in such a way that the population of multiple probes comprises at least one probe for each target sequence that shall become enriched. Preferably, the population of multiple probes additionally comprises at least a second probe for each target sequence that shall become enriched, characterized in that said second probe has a sequence which is complementary to said first sequence.

As an example, the genomic region can be a genomic region representing a gene known to be involved in balanced chromosomal aberrations. Since breakpoints occur both in introns and exons probes may represent the genomic region as complete as possible. Alternatively, to increase the likelihood that desired non-unique or difficult-to-capture targets are enriched, the probes can be directed to sequences associated with (e.g., on the same fragment as, but separate from) the actual target sequence, in which case genomic fragments containing both the desired target and associated sequences will be captured and enriched. The associated sequences can be adjacent or spaced apart from the target sequences, but the skilled person will appreciate that the closer the two portions are to one another, the more likely it will be that genomic fragments will contain both portions. According to the present invention, the captured sequences should be differing from a contiguous genomic region. Hereby, desired target sequences were able to capture a specific nucleic acid molecule. However, in the case of a balanced chromosomal aberration this captured nucleic acid molecule will contain sequences mapping to the corresponding capture probe, but also sequences that are derived from a fusion partner that can be derived from a different part of the genome. As an example, these sequences can be from a different chromosome, e.g. as a result of a translocation (such as t(9;11)(p22;q23)), but also from the same chromosome, e.g. as a result of a inversion (such as inv(16)(p13q22)). Thus, one may consider these chimeric sequences as a possibility to further identify hitherto unknown fusion partner genes.

Still further, to further reduce the limited impact of cross-hybridization by off-target molecules, thereby enhancing the integrity of the enrichment, sequential rounds of capture using distinct but related capture probe sets directed to the target region can be performed. Related probes are probes corresponding to regions in close proximity to one another in the genome that can, therefore, hybridize to the same genomic DNA fragment.

These probes may be either designed to be overlapping probes, meaning that the starting nucleotides of adjacent probes are less than the length of a probe, or non-overlapping probes, where the distance between adjacent probes are greater than the length of a probe. The distance between adjacent probes is generally overlapping, with spacing between the starting nucleotide of two probes varying between 1 and 100 bases. This distance can be varied to cause some genomic regions to be targeted by a larger number of probes than others. This variation can be used to modulate the capture efficiency of individual genomic regions, normalizing capture. Probes can be tested for uniqueness in the genome.

To avoid non-specific binding of genomic elements to capture arrays, highly repetitive elements of the genome should be excluded from selection microarray designs. Therefore, in a preferred embodiment, the oligonucleotide probes do not contain highly repetitive sequences to reduce the likelihood of non-specific binding between the microarrays and genomic nucleic acid molecules. The strategy for identifying and excluding highly-repetitive genomic regions was similar to that of the WindowMasker program (Morgulis et al. (2006) Bioinformatics, 15: 134-141). The average 15-mer frequency of each probe was calculated by comparing the frequencies of all 15-mers present in the probe against a pre-computed frequency histogram of all possible 15-mer probes in the human genome. The likelihood that the probe represents a repetitive region of the genome increases as the average 15-mer frequency increases. Only probes having an average 15-mer frequency below 100 were included on the solid support. Repetitive DNA sequences can also be depleted using a subtraction hybridization protocol as describe by Craig et al. (1997) Hum. Genet., 100: 472- 476).

The solid support according to the present invention is usually a slide, chip or bead, preferably it is either a nucleic acid microarray or a population of beads. Said support may be glass, metal, ceramic and/or polymeric. General immobilization methods include spotting, photolithography or *in situ* synthesis. In case said solid support is a chip or microarray, it is possible to synthesize the oligonucleotide capture probes *in situ* directly onto said solid support. For example, the probes may be synthesized on the microarray using a maskless array synthesizer (see e.g. US 6,375,903). The lengths of the multiple oligonucleotide probes may vary. The probes can be designed for convenient release from the solid support by providing, e.g., at or near the support-proximal probe termini an acid-or alkali-labile nucleic acid sequence that releases the probes under conditions of low or high pH, respectively. Various cleavable linker chemistries are known in the art. The support can be provided, e.g., in a column having fluid inlet and outlet. The art is familiar with methods for immobilizing nucleic acids onto supports, for example by incorporating a biotinylated nucleotide into the probes and coating the support with streptavidin such that the coated support non-covalently attracts and immobilizes the probes in the pool. The length of the linker can range between about 12 and about 100 base pairs, including a range between about 18 and 100 base pairs, and preferably between about 20 and 24 base pairs.

If the solid support is a population of beads, the capture probes may be initially synthesized on a microarray using a maskless array synthesizer, then released or cleaved off according to known standard methods, optionally amplified and then immobilized on said population of beads according to methods known in the art. The beads may be packed into a column so that a sample is loaded and passed through the column for reducing genetic complexity. Alternatively, in order to improve the hybridization kinetics, hybridization may take place in an aqueous solution comprising the beads with the immobilized multiple oligonucleotide molecules in suspension.

In one embodiment, the multiple different oligonucleotide probes each carry a chemical group or linker, i.e. a moiety which allows for immobilization onto a solid support, also named an immobilizable group (see dots on the array in Fig. 2). Then the step of exposing the fragmented, denatured nucleic acid molecules of the sample to the multiple, different oligonucleotide probes under hybridizing conditions is performed in an aqueous solution and immobilization onto an appropriate solid support takes place subsequently. For example, such a moiety may be biotin which can be used for immobilization on a streptavidin coated solid support. In another embodiment, such a moiety may be a hapten like digoxygenin, which can be used for immobilization on a solid support coated with a hapten recognizing antibody, e.g. a digoxygenin binding antibody.

In a specific embodiment, the plurality of immobilized probes is characterized by normalized capture performance. A goal of such normalization is to deliver one gene per read. For example, the number of sequencing reactions required to effectively analyze each target region can be reduced by normalizing the number of copies of each target sequence in the enriched population such that across the set of probes the capture performance of distinct probes are normalized, on the basis of a combination of fitness and other probe attributes. Fitness, characterized by a "capture metric," can be ascertained either informatically or empirically. In one approach, the ability of the target molecules to bind can be adjusted by providing so-called isothermal (Tm-balanced) oligonucleotide probes, as are described in U.S. Published Patent Application No. US2005/0282209, that enable uniform probe performance, eliminate hybridization artifacts and/or bias and provide higher quality output. Probe lengths are adjusted as specified above to equalize the melting temperature (e.g. Tm = 76°C, typically about 55°C to about 76°C, in particular about 72°C to about 76°C) across the entire set. Thus, probes are optimized to perform equivalently at a given stringency in the genomic regions of interest, including AT- and GC-rich regions. Related, the sequence of individual probes can be adjusted, using natural bases or synthetic base analogs such as inositol, or a combination thereof to achieve a desired capture fitness of those probes. Similarly, locked nucleic acid probes, peptide nucleic acid probes or the like having structures that yield desired capture performance can be employed. The skilled artisan in possession of this disclosure will appreciate that probe length, melting temperature and sequence can be coordinately adjusted for any given probe to arrive at a desired capture performance for the probe. Conveniently, the melting temperature (Tm) of the probe can be calculated using the formula: Tm=5x(Gn+Cn)+1x(An+Tn), where n is the number of each specific base (A, T, G or C) present on the probe.

Capture performance can also be normalized by ascertaining the capture fitness of probes in the probe set, and then adjusting the quantity of individual probes on the solid support accordingly. For example, if a first probe captures twenty times as much nucleic acid as a second probe, then the capture performance of both probes can be equalized by providing twenty times as many copies of the second probe, for example by increasing by twenty-fold the number of features displaying the second probe. If the probes are prepared serially and applied to the solid support, the concentration of individual probes in the pool can be varied in the same way.

Still further, another strategy for normalizing capture of target nucleic acids is to subject the eluted target molecules to a second round of hybridization against the probes under less stringent conditions than were used for the first hybridization round. Apart from the substantial enrichment in the first hybridization that reduces complexity relative to the original genomic nucleic acid, the second hybridization can be conducted under hybridization conditions that saturate all capture probes. Presuming that substantially equal amounts of the capture probes are provided on the solid support, saturation of the probes will ensure that substantially equal amounts of each target are eluted after the second hybridization and washing.

Another normalizing strategy follows the elution and amplification of captured target molecules from the solid support. Target molecules in the eluate are denatured using, for example, a chemical or thermal denaturing process, to a single-stranded state and are re-annealed. Kinetic considerations dictate that abundant species re-anneal before less abundant species. As such, by removing the initial fraction of re-annealed species, the remaining single-stranded species will be balanced relative to the initial population in the eluate. The timing required for optimal removal of abundant species is determined empirically.

The normalized capture performance is generally achieved by methods as described above, typically comprising the steps of a) ascertaining the capture fitness of probes in the probe set; and b) adjusting the quantity of at least one probe on the solid support. Alternatively, the normalized capture performance is achieved by a method comprising the steps of a) ascertaining the capture fitness of probes in the probe set; and b) adjusting at least one of the sequence, the melting temperature and the probe length of at least one probe on the solid support. Still alternatively, the normalized capture performance is achieved by a method comprising the steps of a) exposing the captured molecules to the at least one immobilized probe on the solid support under less stringent conditions than in the first exposing step such that the at least one probe is saturated, b) washing unbound and non-specifically bound nucleic acids from the solid support; and c) eluting the bound target nucleic acids from the solid support. Still alternatively, the normalized capture performance is achieved by a method comprising the steps of a) denaturing the eluted captured molecules to a single-stranded state; b) re-annealing the single-stranded molecules until a portion of the molecules are double-stranded; and discarding the double-stranded molecules and c) retaining the single-stranded molecules.

Usually at least one immobilized probe hybridizes to a genomic region of interest on nucleic acid fragments in the sample. Alternatively, the at least one immobilized oligonucleotide probe may hybridize to sequences on target nucleic acid fragments comprising a genomic region of interest, the hybridizing sequences being separate from the genomic region of interest. Furthermore, it is also within the scope of the present invention, that at least a second hybridization step using at least one oligonucleotide probe related to but distinct from the at least one probe used in the initial hybridization is performed.

Advantageously, the method of the present invention further comprises the step of ligating adaptor molecules to one or both, preferably both ends of the fragmented nucleic acid molecules. Adaptor molecules in the context of the present invention are preferably defined as blunt ended double stranded oligonucleotides. In addition, the inventive method may further comprise the step of amplification of said nucleic acid molecules with at least one primer, said primer comprising a sequence which corresponds to or specifically hybridizes with the sequence of said adaptor molecules.

In order to ligate adaptor molecules onto a double stranded target molecule, it is preferred that this target molecule itself is blunt ended. In order to achieve this, the double stranded target molecules are subjected to a fill-in reaction with a DNA polymerase such as T4-DNA polymerase or Klenow polymerase in the presence of desoxynucleoside triphposphates, which results in blunt ended target molecules. In addition, e.g. T4 Polynucleotide kinase is added prior to the ligation in order to add phosphate groups to the 5' terminus for the subsequent ligation step. Subsequent ligation of the adaptors (short double stranded blunt end DNA oligonucleotides with about 3-20 base pairs) onto the polished target DNA may be performed according to any method which is known in the art, preferably by means of a T4-DNA ligase reaction.

Said ligation may be performed prior to or after the step of exposing a sample that comprises fragmented, denatured nucleic acid molecules to multiple, different oligonucleotide probes under hybridizing conditions to capture target nucleic acid molecules that hybridize to said probes. In case ligation is performed subsequently, the enriched nucleic acids which are released from the solid support in single stranded form should be re-annealed first followed by a primer extension reaction and a fill-in reaction according to standard methods known in the art.

Ligation of said adaptor molecules allows for a step of subsequent amplification of the captured molecules. Independent from whether ligation takes place prior to or after the capturing step, there exist two alternative embodiments. In the first embodiment, one type of adaptor molecules is used. This results in population of fragments with identical terminal sequences at both ends of the fragment. As a consequence, it is sufficient to use only one primer in a potential subsequent amplification step. In an alternative embodiment, two types of adaptor molecules A and B are used. This results in a population of enriched molecules composed of three different types: (i) fragments having one adaptor (A) at one end and another adaptor (B) at the other end, (ii) fragments having adaptors A at both ends, and (iii) fragments having adaptors B at both ends.

Generation of enriched molecules according to type (i) is of outstanding advantage, if amplification and sequencing is e.g. performed with the 454 Life Sciences (USA) GS20 and GSFLX instrument (see e.g. GS20 Library Prep Manual, Dec 2006; WO 2004/070007). If one of said adaptors, e.g. adaptor B carries a biotin modification, then molecules (i) and (iii) can e.g. be bound on streptavidin (SA) coated magnetic particles for further isolation and the products of (ii) washed away. In case the enriched and SA-immobilized DNA is single stranded following elution from the capture array/solid support, it is advantageous to make the DNA double-stranded. In this case primers complementary to adaptor A may be added to the washed SA pull down products. Since moieties that are B-B (iii above) do not have A or its complement available, only A-B adapted and SA captured products will be made double stranded following primer-extension from an A complement primer. Subsequently, the double stranded DNA molecules that have been bound to said magnetic particles are thermally or chemically (e.g. with NaOH) denatured in such a way that the newly synthesized strand is released into solution. Due to the tight biotin/streptavidin bonding, for example, molecules with only two adaptors B will not be released into solution. The only strand available for release is the A-complement to B-complement primer-extension synthesized strand. Said solution comprising single stranded target molecules with an adaptor A at one end and an adaptor B at the other end can e.g. subsequently be bound on a further type of beads comprising a capture sequence which is sufficiently complementary to the adaptor A or B sequences for further processing.

The second general step (b) concerns the separation of unbound and non-specifically hybridized nucleic acids from the captured molecules. In some embodiments, the separation can be carried out with means of biotin attached to the captured target nucleic acid molecules. In this case the capture substrate, such as a bead for example a paramagnetic particle, is coated with streptavidin for separation of the target nucleic acid molecule from non-specifically hybridized target nucleic acid molecules. In some embodiments, the captured target nucleic acid molecules are washed prior to elution of the bound or captured molecules.

The next general step (c) concerns the elution of the captured molecules form the solid support, e.g. by an alkaline solution, preferably in an eluate pool which has now a reduced genetic complexity relative to the original sample.

Steps (a) to (c) as well as the intermediate steps as described above can be repeated for at least one further cycle with the eluted captured molecules.

The next general step (e) concerns the determination of the nucleic acid sequence of the captured molecules. Sequencing can be performed by a number of different methods, such as array-based-, shotgun-, capillary-, or other sequencing methods known to the art, preferably by employing sequencing by synthesis technology. Sequencing by synthesis according to the prior art is defined as any sequencing method which monitors the generation of side products upon incorporation of a specific deoxynucleoside-triphosphate during the sequencing reaction (Hyman, 1988, Anal. Biochem. 174:423-436; Rhonaghi et al., 1998, Science 281:363-365).

One particular and most prominent embodiment of the sequencing by synthesis reaction is the pyrophosphate sequencing method. In this case, generation of pyrophosphate during nucleotide incorporation is monitored by means of an enzymatic cascade which finally results in the generation of a chemo-luminescent signal. For example, the 454 Genome Sequencer System (Roche Applied Science Cat. No. 04 760 085 001) is based on the pyrophosphate sequencing technology. Other suitable DNA sequencers are the Genome Analyzer IIx (illumina Inc., San Diego) and the SOLiD™ System (applied biosystems). For sequencing on a 454 GS20 or 454 FLX instrument, the average genomic DNA fragment size should be in the range of 200 or 600 bp, respectively. Alternatively, the sequencing by synthesis reaction is a terminator dye type sequencing reaction. In this case, the incorporated dNTP building blocks comprise a detectable label, which is preferably a fluorescent label that prevents further extension of the nascent DNA strand. The label is then removed and detected upon incorporation of the dNTP building block into the template/primer extension hybrid for example by means of using a DNA polymerase comprising a 3'-5' exonuclease or proofreading activity.

In case of the Genome Sequencer workflow (Roche Applied Science Catalog No. 04 896 548 001), in a first step, (clonal) amplification is performed by emulsion PCR. Thus, it is also within the scope of the present invention, that the step of amplification is performed by emulsion PCR methods. The beads carrying the clonally amplified target nucleic acids may then become arbitrarily transferred into a picotiter plate according to the manufacturer's protocol and subjected to a pyrophosphate sequencing reaction for sequence determination.

The next general step (f) concerns the comparison of the determined sequence to sequences in a database of the reference genome. Such database preferably contains the whole genome or at least one chromosome of an organism, or at least about 90%, in particular at least about 95%, especially at least about 98% of the genome or of at least one chromosome of an organism, in particular a human genome or chromosome. Preferably any primer or adaptor sequence is removed *in silico* prior to this step, i.e. with the help of a suitable computer program, e.g. the gsMapper Version 2.0.01 from Life Sciences, USA.

The next general steps (g) and (h) concern the identification of sequences which only partially match or do not match with sequences of the reference genome. These so-called "unmapped" or only "partially mapped" sequences are of particular interest, since these sequences may contain information on distinct fusion genes as a result of a chromosomal aberration. Generally, as a result of a translocation or inversion, fusion or chimeric genes occur (see Fig. 1). According to the method of the present invention it is sufficient to represent only one fusion partner on the capturing assay since nucleic sequences from the second fusion partner will also be captured by probes of the other fusion partner (see Fig. 2). As such, this method will not only allow detecting known fusion events, but also identify novel fusion partner genes. This is a particular advantage of the present invention because neither cytogenetic nor molecular genetic analyses are able to fully identify or detect balanced chromosomal aberrations or to fully resolve a molecular fusion gene. Moreover, also reciprocal fusion genes can be detected by the present invention. Such sequences are generally identified with the help of a suitable computer program. Consequently, at least one balanced chromosomal aberration can easily be detected in a one-step approach.

An alternative method for detecting balanced chromosomal aberrations in a genome is a method comprising the steps of exposing fragmented, denatured nucleic acid molecules of a target population to multiple, different oligonucleotide probe derived amplicons wherein the amplicons are in solution and wherein the amplicons further comprise a binding moiety, under hybridizing conditions to capture nucleic acid molecules that specifically hybridize to the probe amplicons, binding or capturing the complexes of hybridized molecules by binding the binding moiety found on the probe amplicon to its binding partner, e.g., biotin/SA, digoxigenin/anti-digoxigenin, 6HIS/nickel, etc., separating unbound and non-specifically hybridized nucleic acids from the bound probe amplicons, eluting the hybridized target molecules from the amplicons, and sequencing the target molecules. In detail, the method comprises the following steps:
(a) providing:
   i) a solid support comprising multiple, different oligonucleotide probes located on multiple, different sites of the solid support, wherein said oligonucleotide probes have an average size of about 20 to about 100 nucleotides, preferably about 40 to about 85 nucleotides, more preferred about 45 to about 75 nucleotides, in particular about 55 to about 65 nucleotide residues or about 60 nucleotide residues,
   ii) a nucleic acid sample comprising target nucleic acid molecules,
(b) amplifying said oligonucleotide probes wherein the amplification products comprise a binding moiety and wherein said amplification products are maintained in solution,
(c) hybridizing the target nucleic acid molecules to said amplification products in solution under specific hybridizing conditions, wherein, prior to hybridization, the target nucleic acid molecules are fragmented and denatured and have an average size of about 100 to about 1000 nucleotide residues, preferably about 250 to about 800 nucleotide residues and most preferably about 400 to about 600 nucleotide residues, in particular about 500 nucleotide residues,
(d) separating the hybridization complexes of target nucleic acid molecules and amplification products from non-specifically hybridized nucleic acids by said binding moiety,
(e) separating the target nucleic acid molecules from the complex,
(f) determining the nucleic acid sequence of the separated target nucleic acid molecules, in particular by means of performing sequencing by synthesis reactions,
(g) comparing the determined sequence to sequences in a database of the reference genome,
(h) identifying sequences in the determined sequence which only partially match or do not match with sequences of the reference genome,
(i) detecting at least one balanced chromosomal aberration.

According to this method, the target nucleic acid molecules are hybridized with oligonucleotide probes containing a binding moiety in solution in order to reduce the complexity of the target nucleic acids molecules as described in WO2009/053039. Steps (f) to (i) of the alternative method are the same as steps (e) to (h) of the first embodiment of the present invention as described above. Therefore, the above-specified features also apply for the alternative method outlined above.

In a preferred embodiment, the multiple, different oligonucleotide probes each contain a chemical group or linker being able to bind to a solid support, as described above. Furthermore, the fragmented target nucleic acid molecules may further comprise adaptor molecules at one or both ends, as described above. In addition, the oligonucleotide probes may further comprise primer binding sequences at one or both ends of said probes, whereas when present at both ends of the probes the primer binding sequences may be the same or be different, as also described above. The length of the linker can range between about 12 and about 100 base pairs, including a range between about 18 and 100 base pairs, and preferably between about 20 and 24 base pairs. Adaptor molecules in the context of the present invention are preferably defined as blunt-ended double-stranded oligonucleotides.

Generally, the amplification reaction comprises exponential polymerase chain reaction, in particular exponential polymerase chain reaction and further asymmetric polymerase chain reaction.

In another preferred embodiment, the binding moiety is a biotin binding moiety, wherein said separating preferably comprises binding said biotin binding moiety to a streptavidin coated substrate, in particular to a straptavidin coated paramagnetic particle.

The solution comprising the probe derived amplicons is transferred to, for example, a tube, well, or other vessel and maintained in solution. It is contemplated that one or more additional rounds of amplification to boost the production of the amplicon strand that comprises the binding moiety, for example by asymmetric PCR, is additionally performed. A nucleic acid sample, fragmented and denatured to yield fragmented single stranded target sequences, is added to the amplicons in solution and hybridization is allowed to occur between the probe derived amplicons and the fragmented single stranded target nucleic acid sample. After hybridization, nucleic acids that do not hybridize, or that hybridize non-specifically, are separated from the amplicon/target complex by capturing the amplicon/target complex via the binding moiety and washing the amplicon/target complex. For example, if the binding moiety is biotin, a streptavidin coated substrate is used to capture the complex. The bound complex is washed, for example with one or more washing solutions. The remaining nucleic acids (e.g., specifically bound to the amplicons) are eluted from the complex, for example, by using water or an elution buffer (e.g., comprising TRIS buffer and/or EDTA) to yield an eluate enriched for the target nucleic acid sequences.

Therefore, the present method preferably further comprises washing said hybridization complexes prior to separating the target nucleic acid molecules from the complex. The method may also further comprise the step of amplification the separated target nucleic acid molecule prior to step (f), in particular by emulsion polymerase chain reaction.

As also described above, the nucleic acid molecules are generally genomic DNA molecules, in particular containing the whole genome, at least one gene of an organism or at least one chromosome of an organism, or at least one genomic nucleic acid molecule with a size of at least about 50 kb, at least about 200 kb, at least about 500 kb, at least about 1 Mb, at least about 2 Mb or at least about 5 Mb, especially a size between about 100 kb and about 5 Mb, between about 200 kb and about 5 Mb, between about 500 kb and about 5 Mb, between about 1 Mb and about 2 Mb or between 2 Mb and about 5 Mb.

As also described above, the oligonucleotide probes contain exons, introns and/or regulatory sequences from at least a part of a genome of an organism, having a size of at least 50 kb, in particular of at least 100 kb, preferably at least 1 Mb or at least one of the sizes as specified above, or from at least one gene or at least one chromosome of an organism, preferably from at least about 90%, in particular at least about 95%, especially at least about 98% of the gene or genome of an organism, in particular a human gene or genome.

Again, probes with highly repetitive sequences may also be excluded as described above and the database of the reference genome may contain the whole genome or at least one chromosome of an organism, or at least about 90%, in particular at least about 95%, especially at least about 98% of the genome or of at least one chromosome of an organism, in particular a human genome or chromosome.

Preferably, any primer or adaptor sequence is removed *in silico* prior to step (f) and the solid support may either a nucleic acid microarray or a population of beads. Other solid supports are also possible and already described above.

Taken together, the present invention as described above is generally useful in searching for balanced chromosomal aberrations. Hereby, the invention is useful in a methodology that captured sequences are at least at some point also differing from a known contiguous genomic region. Hereby, desired target sequences were able to capture a specific nucleic acid molecule. However, in the case of a balanced chromosomal aberration this captured nucleic acid molecule will contain sequences mapping to the corresponding capture probe, but also sequences that are derived from a fusion partner that can be derived from a different part of the genome. As an example, these sequences can be from a different chromosome, e.g. as a result of a translocation (such as t(9; 11)(p22;q23)), but also from the same chromosome, e.g. as a result of an inversion (such as inv(16)(p13q22)). Thus, one may consider these chimeric sequences as a possibility to further identify hitherto unknown fusion partner genes resulting from balanced chromosomal aberrations. Generally, the present invention is also directed to the detection, characterization, sub-type classification and/or optimal treatment of diseases, in particular malignancies like lymphomas or leukemias, especially AML.

More important, the present invention enables that fusion genes, point mutations, as well as deletions and insertions can be detected in a one-step approach. Such genetic characterization enables the detection and/or an optimal treatment of diseases, in particular malignancies like lymphomas or leukemias, especially AML.

Consequently, the present invention is also directed to the detection of at least one further mutation, in particular at least one further deletion, especially a deletion in the breakpoint area of the translocation or inversion, at least one further insertion and/or at least one further substitution in the genome, e.g. at least one single nucleotide polymorphism (SNP).

### FIGURES

- Fig. 1: schematically shows an example of the chromosomal translocation AML with t(9;11)(p22;q23) and molecular fusion of *MLL-MLLT3.* On the molecular level *MLL* (HGNC: 7132) is fused to the partner gene *MLLT3* (HGNC: 7136) from chromosome 9.
- Fig. 2: schematically shows the capturing of both MLL sequences and chimeric nucleic acids according to Example 2. Chip probes are designed to capture nucleic acids of a gene of interest, e.g. *MLL* on chromosome 11, q23). Capture probes hybridize against the *MLL* gene, but as a result of a translocation, other sequences are also attached. Consequently, this nucleic acid molecule has chimeric properties, i.e. it contains gene information for a first gene, here *MLL,* and from a second gene, the molecular fusion partner gene, here *MLLT3.*

### EXAMPLES

The following examples are provided in order to demonstrate and further illustrate certain preferred embodiments and aspects of the present invention and are not to be construed as limiting the scope thereof.

### 1. Generic Example

This generic example describes how to perform selection that allows for rapid and efficient characterization of balanced chromosomal aberrations, such as translocations or inversions, occurring in particular in cancer genomes. Moreover, this generic example describes the method how to discover a hitherto unknown fusion partner gene. Microarrays having immobilized probes are used in one- or multiple rounds of hybridization selection with a target of total genomic DNA, and the selected sequences are amplified by LM-PCR. All laboratory steps are principally based on the NimbleGen User Guide Version 3.1, 7 Jul (2009).

### a) Preparation of the genomic DNA and double-stranded linkers

DNA is fragmented using nebulization to an average size of ∼500 base pairs. A reaction to polish the ends of the nebulized DNA fragments is set up:

### Polishing Master Mix

| | |
|---|---|
| 10X NEB T4 DNA Polymerase Buffer (NEB2) | 12µl |
| water | 9µl |
| 100X NEB BSA | 1µl |
| 25mM dNTP stock (mixing 100µl each of 100mM dA, dC, dT, and dGTP) | 5µl |
| 100mM ATP (ribonucleotide) | 1µl |
| 3U/µl T4 DNA Polymerase | 6µl |
| 10U/µl T4 Polynucleotide Kinase | 6µl |
| Total | 40µl |

The reaction is incubated at 20 minutes at 12°C, 20 minutes at 25°C and 20 minutes at 75°C. The reaction is then subjected to linker ligation. Two complementary oligonucleotides are annealed to create a double-stranded linker, by mixing the following:
gSel3 = 5' - CTC GAG AAT TCT GGA TCC TC - 3'
gSel4-Pi = 5' - Phos/GAG GAT CCA GAA TTC TCG AGT T - 3'

In 0.2ml strip tubes, 5µl of 4,000µM gSel3 with 5µl of 4,000µM gSel4-Pi are mixed. As many tubes as possible are prepared with the synthesized primers. Each tube now contains 2,000µM of each linker.

The PCR reaction is performed as follows: 95°C for 5 minutes, ramp cool 0.1°C per second to 12°C, and hold at 12°C. Using Oligo Annealing Buffer (OAB) a solution is created containing 500µM working stock of linkers.

### Oligo Annealing Buffer (OAB)

| | |
|---|---|
| 1M Tris-HCl (pH 7.8) | 100µl |
| 0.5M EDTA (pH 8.0) | 20µl |
| 5M NaCl | 100µl |
| VWR water | 9.78ml |
| Total | 10ml |

The length of the 2 complementary oligonucleotides 1 and 2 is between 12 and 24 nucleotides, and the sequence is selected depending upon the functionality desired.

### b) Ligation of linkers to genomic DNA fragments

The following reaction to ligate the linkers to genomic DNA fragments is set up: to 5 µg of polished DNA, 8µl of 500µM annealed linker stock are added:

### Ligation Master Mix

| | |
|---|---|
| 10X NEB Buffer 2 | 8µl |
| 100mM ATP (ribonucleotide) | 4µl |
| VWR water | 51µl |
| T4 DNA Ligase | 10µl |
| Total | 73µl |

The ligation reaction is incubated in a thermocycler at 25°C for 90 minutes. Ligated genomic DNA is subsequently purified in order to remove small fragments.

### c Primary selection and capture of hybrids

To prepare the genomic DNA sample for hybridization to the microarray, linker modified genomic DNA (5µg) is resuspended in 4.8µl of nuclease-free water and combined with 8.0µl. NimbleGen Hybridization Buffer (Roche NimbleGen, Inc., Madison, WI), 3.2µl Hybridization Additive (Roche NimbleGen, Inc), in a final volume of 16µl. The samples are heat-denatured at 95°C for 5 minutes and transferred to a 42°C heat block.

To capture the target genomic DNA on the microarray, samples are hybridized to NimbleGen CGH arrays, manufactured as described in US 6,375,903 (Roche NimbleGen, Inc.). Maskless fabrication of capture oligonucleotides on the microarrays is performed by light-directed oligonucleotide synthesis using a digital micromirror as described in Singh-Gasson et al. (1999, Nat. Biotech. 17:974-978) as performed by a maskless array synthesizer. Gene expression analysis using oligonucleotide arrays produced by maskless photolithography is described in Nuwaysir et al. (2002, Genome Res. 12:1749-1755). Hybridization is performed in a MAUI Hybridization System (BioMicro Systems, Inc., Salt Lake City, UT) according to manufacturer instructions for 72 hours at 42°C using mix mode B. Following hybridization, arrays are washed twice with Wash Buffer I (0.2x SSC, 0.2% (v/v) SDS, 0.1mM DTT, NimbleGen Systems) for a total of 2.5 minutes. Arrays are then washed for 1 minute in Wash Buffer II (0.2x SSC, 0.1mM DTT, NimbleGen Systems) followed by a 15 second wash in Wash Buffer III (0.05x SSC, 0.1mM DTT, Roche NimbleGen, Inc.). To elute the genomic DNA hybridized to the microarray, the arrays are washed with 425µl of the 125mM NaOH solution using an elution chamber. The eluted DNA then is purified (Qiagen MinElute column protocol).

### d) Amplification of the primary selected DNA

The primary selected genomic DNA is amplified as described below. Twelve separate replicate amplification reactions are set up. Only one oligonucleotide primer is required because each fragment has the same linker ligated to each end:
Reaction reagents: final total volume of 50 µl
Template: primary selection material 25 µl

### LM-PCR Master Mix Amount

| | |
|---|---|
| 10X ThermoPol Reaction Buffer | 4.9µl |
| 25mM dNTP | 0.5µl |
| 40µM gSel3 | 6.25µl |
| VWR water | 11.35µl |
| 5U/µl Taq DNA Polymerase | 1µl |
| 0.05U/µl PfuTurbo DNA Polymerase | 1µl |
| Total | 25µl |

The reactions are amplified according to the following program:

| Cycle number | Denaturation | Annealing | Polymerization |
|---|---|---|---|
| 1 | 2 min at 95°C | | |
| 2-28 | 1 min at 95°C | 1 min at 60°C | 2 min at 72°C |

followed by a final elongation of 5 min at 72°C. The amplification products are purified using a QIAquick PCR purification kit. The eluted samples are pooled and the concentration of amplified primary selected DNA is determined by spectrophotometry.

### Example 2: Detection of Balanced Chromosomal Aberrations of AML

DNA sequence enrichment from complex genomic samples using microarrays and a 454 PicoTiterPlate (PTP) pyrosequencing assay with long-oligonucleotide sequence capture arrays was applied to allow a comprehensive genetic characterization in a one-step procedure. Three AML cases were analyzed with either known chromosomal aberrations-inversions and translocations - leading to fusion genes (*CBFB-MYH11, MLL-MLLT3, MLL-unidentified fusion partner)* according to the experimental conditions explained in the generic example (Example 1).

A high-density oligonucleotide microarray that captured short segments that correspond to 92 individual gene exon regions (approximately 1.91Mb of total sequence, sequence build HG18) was synthesized according to standard Roche NimbleGen, Inc (385K format; Madison, WI). microarray manufacturing protocols. Overlapping microarray probes of more than 60 bases each on the array spanned each target genome region, with a probe positioned each 10 bases for the forward strand of the genome. In addition, full genomic regions were represented for three additional target genes *(MLL, RUNX1, CBFB).*

To test the performance of the capture system, the genomic design was first used to capture fragmented genomic DNA from an acute myeloid leukemia (AML) patient sample (case N1). This case harboured an inv(16)(p13q22) aberration, as confirmed by cytogenetics. On a molecular level the *CBFB* gene is fused to *MYH11* on chromosome 16. A second case (N3) was characterized by a translocation with a confirmed known partner gene (fusion between *MLL* and *MLLT3).* This patient harboured a translocation t(9;11)(p22;q23). A third case was analyzed that was known to have a rearrangement of chromosomal material with involvement of the cytoband 11q23. However, neither cytogenetic nor molecular genetic analyses were able to fully identify the balanced chromosomal aberration or to fully resolve a molecular fusion gene. This genomic region is frequently involved in rearrangements. The MLL gene is known to have many partner genes. The majority of partner genes is known but several genomic loci are not yet fully characterized (Meyer C et al., Leukemia. 2009 Aug;23(8):1490-9.). As such, this case N5 can be characterized as *MLL-X* where the unknown partner gene was suspected to be located on chromomal band 19p13.1.
Case N1: AML with inv(16)(p13q22) and molecular fusion of *CBFB-MYH11*
Case N3: AML with t(9;11)(p22;q23) and molecular fusion of *MLL-MLLT3*
Case N5: AML with t(11q23)/*MLL* and unknown partner gene fused to *MLL*

Briefly, genomic DNA (20 µg) was subjected to nebulization. 5 µg of the fragmented DNA was processed according to the standard NimbleGen laboratory workflow, i.e. polishing, linker ligation. The linker-terminated fragments were denatured to produce single stranded products that were exposed to the capture microarrays under hybridization conditions in the presence of 1X hybridization buffer (Roche NimbleGen, Inc.) for approximately 72 hours at 42°C with active mixing using a MAUI hybridization station (Roche NimbleGen, Inc.). Single-stranded molecules that did not hybridize were washed from the microarrays under stringent washing conditions, 3 x 5 minutes with Stringent Wash Buffer (Roche NimbleGen, Inc.) and rinsed with Wash Buffers I, II, and III (Roche NimbleGen, Inc.). Fragments captured on the microarrays were immediately eluted with 125 mM NaOH and processed for amplification by LM-PCR using a primer complementary to the previously ligated linker oligonucleotides.

To quantify enrichment of the sample genomic DNA, four regions were selected for quantitative PCR (qPCR). These regions were amplified using the following primers (Primer Sequences (5' → 3'). These assays act as a proxy for estimating the enrichment of larger populations of capture targets without a need for sequencing. If qPCR analysis using NSC assays indicates a successful capture of the control loci, it is likely that the experimental loci of interest were also successfully captured.
- NSC-0237: F: CGCATTCCTCATCCCAGTATG (SEQ ID NO:3)
R: AAAGGACTTGGTGCAGAGTTCAG (SEQ ID NO:4)
- NSC-0247: F: CCCACCGCCTTCGACAT (SEQ ID NO:5)
R: CCTGCTTACTGTGGGCTCTTG (SEQ ID NO:6)
- NSC-0268: F: CTCGCTTAACCAGACTCATCTACTGT (SEQ ID NO:7)
R: ACTTGGCTCAGCTGTATGAAGGT (SEQ ID NO:8)
- NSC-0272: F: CAGCCCCAGCTCAGGTACAG (SEQ ID NO:9)
R: ATGATGCGAGTGCTGATGATG (SEQ ID NO:10)

After a single round of microarray capture, the enriched and LM-PCR amplified samples were compared against the non-enriched and LM-PCR amplified samples (i.e. not hybridized to a capture array) using a LighCyclerLC480 real-time PCR system (Roche Applied Science, Mannheim, Germany) measuring SYBR green fluorescence according to manufacturer's protocols. In detail, 218-fold (case N1), 172-fold (case N3), and 281-fold (case N5) enrichment was achieved for the three AML samples. The theoretical maximum enrichment level was 600 fold (3,000 Mb in the genome and 5 Mb of total sequence).

Samples eluted from the capture microarrays were ligated to 454-sequencing-compatible linkers, amplified using emulsion PCR on beads and sequenced using the 454 FLX sequencing instrument Titanium chemistry workflow (454, Branford CT). DNA sequencing of the three samples on the 454 FLX instrument generated 84.0 Mb (case N1), 54.8 Mb (case N3), and 65.8 Mb of total sequence (case N5), respectively. Individual reads were as follows: case N1: 252,651 sequencing reads, case N3: 167,233 reads, case N5: 211,114 reads, respectively.

Following *in silico* removal of the linker sequence (e.g. with the gsMapper Version 2.0.01 from Life Sciences, USA.), each sequencing read was compared to the entire appropriate version of the Human Genome using BLAST analysis (Altschul, et al., 1990, J. Mol. Biol. 215:403-410) using a cut-off score of e = 10⁻⁴⁸, tuned to maximize the number of unique hits. Captured sequences that, according to the original BLAST comparison, map uniquely back to regions within the target regions were considered sequencing hits. These were then used to calculate the % of reads that hit target regions, and the fold sequencing coverage for the entire target region. Data was visualized using SignalMap software (Roche NimbleGen, Inc.). BLAST analysis showed that 88.7% (case N1), 89.6% (case N3), and 88.4% (case N5)of reads, respectively, mapped back uniquely to the genome; 61.4% (case N1), 65.5% (case N1), and 80.5% (case N1) were from targeted regions. The median per-base coverage for each sample was 22.8-fold (case N1), 15.9- (case N3) and 24.1-fold coverage (case N3), respectively (Table 1).

**Table 1:**

| DNA Sample | qPCR Fold Enrichment | FLX-Yield (Mb) | Percentage of Reads Mapped Uniquely to the Genome | Percentage of Total Reads That Mapped to Selection Targets | Median Fold Coverage for Target Regions |
|---|---|---|---|---|---|
| N1 | 218 | 80.8 | 88.6% | 61.4% | 22.8 |
| N3 | 172 | 53.0 | 89.6% | 65.5% | 15.9 |
| N5 | 281 | 64.0 | 88.4% | 80.5% | 24.1 |

Reads that did not uniquely map back to the reference genome were not discarded, but were analyzed further for the detection of chimeric reads. As such, reads that were "partially" mapped to the reference genome, or reads that were discovered to not map to the genome were further filtered to detect chimeric sequences.

### Case N1

Here a total of 13 reads was observed to carry sequence information that map both to the MYH11 gene and the CBFB gene. For example sequence read No. 1 of Table 1 has a total length of 449 bases. Of these bases 1 - 240 map to chromosome 16 (Start: 15,722,449; End: 15,722,688). Of these 242 bases, 230 were found to be representative for MYH11. The remainder sequence of the bases 241 to 449 was detected to represent the CBFB gene with chromosome 16 Start at 65,678,383and End at 65,678,590.

**Table 2:**

| No. | Start | End | Length | Location | Base start | Base end | Identity | Gene Symbol |
|---|---|---|---|---|---|---|---|---|
| 1 | 1 | 240 | 449 | chr16 | 15,722,449 | 15,722,688 | (230/242 ident) | ***MYH11*** |
| | 449 | 241 | 449 | chr16 | 65,678,383 | 65,678,590 | (205/210 ident) | ***CBFB*** |
| 2 | 62 | 492 | 492 | chr16 | 15,722,691 | 15,723,119 | (428/431 ident) | ***MYH11*** |
| | 61 | 20 | 492 | chr16 | 65,678,590 | 65,678,631 | (42/42 ident) | ***CBFB*** |
| 3 | 509 | 287 | 509 | chr16 | 15,722,468 | 15,722,690 | (220/225 ident) | ***MYH11*** |
| | 1 | 286 | 509 | chr16 | 65,678,301 | 65,678,588 | (276/289 ident) | ***CBFB*** |
| 4 | 181 | 1 | 326 | chr16 | 15,722,692 | 15,722,865 | (169/181 ident) | ***MYH11*** |
| | 182 | 326 | 326 | chr16 | 65,678,589 | 65,678,734 | (142/146 ident) | ***CBFB*** |
| 5 | 1 | 233 | 461 | chr16 | 15,722,452 | 15,722,688 | (224/237 ident) | ***MYH11*** |
| | 441 | 234 | 461 | chr16 | 65,678,382 | 65,678,590 | (205/210 ident) | ***CBFB*** |
| 6 | 266 | 1 | 463 | chr16 | 15,722,692 | 15,722,952 | (254/266 ident) | ***MYH11*** |
| | 267 | 463 | 463 | chr16 | 65,678,589 | 65,678,783 | (193/197 ident) | ***CBFB*** |
| 7 | 356 | 1 | 431 | chr16 | 15,722,692 | 15,723,049 | (347/360 ident) | ***MYH11*** |
| | 357 | 411 | 431 | chr16 | 65,678,589 | 65,678,643 | (55/55 ident) | ***CBFB*** |
| 8 | 159 | 475 | 475 | chr16 | 15,722,691 | 15,723,004 | (313/317 ident) | ***MYH11*** |
| | 158 | 20 | 475 | chr16 | 65,678,590 | 65,678,726 | (135/139 ident) | ***CBFB*** |
| 9 | 1 | 142 | 502 | chr16 | 15,722,553 | 15,722,688 | (134/142 ident) | ***MYH11*** |
| | 502 | 143 | 502 | chr16 | 65,678,232 | 65,678,590 | (354/362 ident) | ***CBFB*** |
| 10 | 198 | 489 | 489 | chr16 | 15,722,691 | 15,722,983 | (291/293 ident) | ***MYH11*** |
| | 197 | 1 | 489 | chr16 | 65,678,590 | 65,678,779 | (186/197 ident) | ***CBFB*** |
| 11 | 1 | 241 | 356 | chr16 | 15,722,449 | 15,722,688 | (230/243 ident) | ***MYH11*** |
| | 356 | 242 | 356 | chr16 | 65,678,479 | 65,678,590 | (111/116 ident) | ***CBFB*** |
| 12 | 295 | 1 | 516 | chr16 | 15,722,692 | 15,722,984 | (287/297 ident) | ***MYH11*** |
| | 296 | 516 | 516 | chr16 | 65.678.589 | 65,678,811 | (221/223 ident) | ***CBFB*** |
| 13 | 333 | 508 | 508 | chr16 | 15,722,691 | 15,722,866 | (176/176 ident) | ***MYH11*** |
| | 332 | 1 | 508 | chr16 | 65,678,590 | 65,678,918 | (323/333 ident) | ***CBFB*** |

### Case N3

Here a total of 8 reads was observed to carry sequence information that map both to the *MLL* gene and the *MLLT3* gene. For example sequence read No. 1 of Table 3 has a total length of 436 bases. Of these bases 296 - 436 map to chromosome 11 (Start: 117859810; End: 117859950). Of these 141 bases, 140 were found to be representative for *MLL.* The remainder sequence of the bases 1 to 194 was detected to represent the *MLLT3* gene with chromosome 11 Start at 20,350,483 and End at 20,350,776.

**Table 3:**

| No. | Start | End | Length | Location | Base start | Base end | Identity | Gene Symbol |
|---|---|---|---|---|---|---|---|---|
| 1 | 436 | 296 | 436 | chr11 | 117859810 | 117859950 | (140/141 ident) | ***MLL*** |
| | 1 | 294 | 436 | chr9 | 20350483 | 20350776 | (294/294 ident) | ***MLLT3*** |
| 2 | 164 | 1 | 401 | chr11 | 117859951 | 117860114 | (164/164 ident) | ***MLL*** |
| | 168 | 401 | 401 | chr9 | 20350778 | 20351012 | (233/235 ident) | ***MLLT3*** |
| 3 | 163 | 1 | 278 | chr11 | 117859951 | 117860113 | (163/163 ident) | ***MLL*** |
| | 167 | 278 | 278 | chr9 | 20350778 | 20350889 | (112/112 ident) | ***MLLT3*** |
| 4 | 91 | 425 | 425 | chr11 | 117859951 | 117860281 | (330/336 ident) | ***MLL*** |
| | 87 | 1 | 425 | chr9 | 20350778 | 20350864 | (87/87 ident) | ***MLLT3*** |
| 5 | 270 | 490 | 490 | chr11 | 117859951 | 117860173 | (220/223 ident) | ***MLL*** |
| | 266 | 1 | 490 | chr9 | 20350778 | 20351043 | (266/266 ident) | ***MLLT3*** |
| 6 | 480 | 345 | 480 | chr11 | 117859815 | 117859950 | (135/136 ident) | ***MLL*** |
| | 1 | 343 | 480 | chr9 | 20350432 | 20350776 | (343/345 ident) | ***MLLT3*** |
| 7 | 237 | 492 | 492 | chr11 | 117859951 | 117860208 | (255/258 ident) | ***MLL*** |
| | 233 | 1 | 492 | chr9 | 20350778 | 20351010 | (233/233 ident) | ***MLLT3*** |
| 8 | 62 | 381 | 381 | chr11 | 117859951 | 117860270 | (318/322 ident) | ***MLL*** |
| | 58 | 1 | 381 | chr9 | 20350778 | 20350835 | (58/58 ident) | ***MLLT3*** |

### Case N5

In this case, a translocation t(11;19)(q23;p13) had been observed in chromosome banding analysis and the involvement of the *MLL* gene had been proven by fluorescence in situ hybridization. However, using RT-PCR no fusion transcripts could be amplified. In contrast, the next-generation sequencing approach identified chimeric reads.

Here a total of 5 reads was observed to carry sequence information that map to the *MLL* gene. For example sequence read No. 1 of Table 5 has a total length of 480 bases. Of these bases 310 - 480 map to chromosome 11 (Start: 117860271; End: 117860441). Of these 173 bases, 169 were found to be representative for *MLL.* The remainder sequence of the bases 1 to 309 was detected to represent the *ELL* gene with chromosome 19 Start at 18430871 and End at 18431174 (identity was 299/309 bases). Since in this case both cytogenetic analysis and molecular PCR-based assays failed to reveal the partner gene, the present invention is a useful method to detect any fusion partner gene. It is sufficient to capture one partner gene, in this case *MLL,* and to capture and subsequently sequence any occurring chimeric reads.

This is illustrated by additional chimeric reads which were composed of *SFRS14* (splicing factor, arginine/serine-rich 14; also located on 19p13 centromeric of ELL) and *MLL.* This suggested that a deletion had occurred in the breakpoint area and thus prevented the formation of a reciprocal *ELL-MLL* fusion gene. SNP array analysis (Affymetrix genome-wide human SNP array 6.0) were performed and data from the SNP microarrays demonstrated a 615 kb deletion on 19p13, flanked by *ELL* and *SFRS14,* spanning from chr19: 18,346,048 - 18,961,490. As such, a microdeletion was causative for the fusion of *SFRS14* to *MLL* in the reciprocal setting.

**Table 4:**

| No. | Start | End | Length | Location | Base start | Base end | Identity | Gene Symbol |
|---|---|---|---|---|---|---|---|---|
| 1 | 480 | 310 | 480 | chr11 | 117860271 | 117860441 | (169/173 ident) | ***MLL*** |
| | 1 | 309 | 480 | chr19 | 18430871 | 18431174 | (299/309 ident) | ***ELL*** |
| 2 | 414 | 373 | 414 | chr11 | 117860400 | 117860441 | (42/42 ident) | ***MLL*** |
| | 1 | 372 | 414 | chr19 | 18430807 | 18431174 | (361/374 ident) | ***ELL*** |
| 3 | 123 | 471 | 471 | chr11 | 117860460 | 117860809 | (343/351 ident) | ***MLL*** |
| | 122 | 9 | 471 | chr19 | 18962849 | 18962964 | (110/116 ident) | ***SFRS14*** |
| 4 | 395 | 458 | 458 | chr11 | 117860460 | 117860523 | (64/64 ident) | ***MLL*** |
| | 394 | 1 | | chr19 | 18962849 | 18963236 | (383/394 ident) | ***SFRS14*** |
| 5 | 123 | 418 | 418 | chr11 | 117860460 | 117860752 | (291/296 ident) | ***MLL*** |
| | 122 | 9 | 418 | chr19 | 18962849 | 18962964 | (109/116 ident) | ***SFRS14*** |

These data illustrate the advantages of the present invention. A programmable high-density array platform with 385,000 probes was used. The probes were readily able to capture up to 5 Mb of total sequence. In addition, to the specificity of the assay, the high yields of the downstream DNA sequencing steps are consistently superior to the routine average performance using non-captured DNA sources. This is attributed to the capture-enrichment process providing a useful purification of unique sequences away from repeats and other impurities that can confound, for example, the first emulsion PCR step of the 454 sequencing process.

In the present example, a computer program was used to map the obtained reads both exactly against the human genome, but also searched for chimeric sequences mapping to different regions in the genome. By this approach all corresponding fusion genes in our examples were detected as *CBFB-MYH11* as well as the reciprocal *MYH11-CBFB* and *MLL-MLLT3* and *MLLT3-MLL,* respectively. It was further demonstrated that fusion genes can be detected in a one-step methodological approach using the combination of a targeted DNA sequence enrichment assay followed by next-generation sequencing technology. In this embodiment, the genomic representation of only one of the partner genes of a chimeric fusion on this capture platform is sufficient to identify also any potentially unknown partner gene as a result of a balanced chromosomal aberration. Moreover, also reciprocal fusion constructs will be revealed. As such, this novel assay has a strong potential to become an important method for a comprehensive genetic characterization of particularly leukemias and other malignancies.

## Claims

1. A method for detecting balanced chromosomal aberrations in a genome, the method comprising the steps of:
(a) exposing fragmented, denatured nucleic acid molecules of said genome to multiple, different oligonucleotide probes containing exons, introns and/or regulatory sequences from at least a part of a genome of an organism having a size of at least 50 kb or from at least one gene or at least one chromosome of an organism, wherein the oligonucleotide probes are located on multiple, different sites of a solid support under hybridizing conditions to capture nucleic acid molecules that specifically hybridize to said probes,
wherein said fragmented, denatured nucleic acid molecules have an average size of about 100 to about 1000 nucleotide residues, preferably about 250 to about 800 nucleotide residues and most preferably about 400 to about 600 nucleotide residues, in particular about 500 nucleotide residues,
wherein said oligonucleotide probes have an average size of about 20 to about 100 nucleotides, preferably about 40 to about 85 nucleotides, more preferred about 45 to about 75 nucleotides, in particular about 55 to about 65 nucleotide residues or about 60 nucleotide residues,
(b) separating unbound and non-specifically hybridized nucleic acids from the captured molecules;
(c) eluting the captured molecules from the solid support,
(d) optionally repeating steps (a) to (c) for at least one further cycle with the eluted captured molecules,
(e) determining the nucleic acid sequence of the captured molecules by means of performing sequencing by synthesis reactions,
(f) comparing the determined sequence to sequences in a database of the reference genome,
(g) identifying sequences in the determined sequence which do not uniquely map back to the reference genome for the identification of a chimeric sequence, wherein the chimeric sequence maps to different regions in the genome, and
(h) detecting at least one balanced chromosomal aberration.

2. The method according to claim 1, wherein the multiple, different oligonucleotide probes each contain a chemical group or linker being able to bind to a solid support.

3. The method according to claim 1 or 2, further comprising the step of ligating adaptor molecules to one or both, preferably both ends of the nucleic acid molecules prior or after the step (a);
particularly further comprising the step of amplification of said nucleic acid molecules with at least one primer, said primer comprising a sequence which specifically hybridizes to the sequence of said adaptor molecule(s), wherein the step of amplification is carried out after step (c) or after step (d).

4. The method according to at least one of the claims 1 to 3, further comprising the step of purifying said nucleic acid molecules prior to step (e);
particularly further comprising the step of amplification the purified nucleic acid molecule prior to step (e), in particular by emulsion polymerase chain reaction.

5. The method according to at least one of the claims 1-4, wherein said nucleic acid molecules are genomic DNA molecules, in particular containing the whole genome or at least one chromosome of an organism, or at least one genomic nucleic acid molecule with a size of at least about 50 kb, at least about 200 kb, at least about 500 kb, at least about 1 Mb, at least about 2 Mb or at least about 5 Mb, especially a size between about 100 kb and about 5 Mb, between about 200 kb and about 5 Mb, between about 500 kb and about 5 Mb, between about 1 Mb and about 2 Mb or between 2 Mb and about 5 Mb.

6. The method according to at least one of the claims 1-5, wherein the oligonucleotide probes containing exons, introns and/or regulatory sequences from at least a part of a genome of an organism, have a size of at least 100 kb, preferably at least 1 Mb or at least one of the sizes as specified in claim 8, or wherein the oligonucleotide probes contain exons, introns and/or regulatory sequences from at least about 90%, in particular at least about 95%, especially at least about 98% of the gene or genome of an organism, in particular a human gene or genome.

7. The method according to at least one of the claims 1-6, wherein
i) probes with highly repetitive sequences are excluded;
ii) the database of the reference genome contains the whole genome or at least one chromosome of an organism, or at least about 90%, in particular at least about 95%, especially at least about 98% of the genome or of at least one chromosome of an organism, in particular a human genome or chromosome;
iii) any primer or adaptor sequence is removed *in silico* prior to step (f);
and/or
iv) said solid support is either a nucleic acid microarray or a population of beads.

8. A method for detecting balanced chromosomal aberrations in a genome, the method comprising the steps of:
(a) providing:
i) a solid support comprising multiple, different oligonucleotide probes containing exons, introns and/or regulatory sequences from at least a part of a genome of an organism having a size of at least 100 kb or from at least one chromosome of an organism, wherein the oligonucleotide probes are located on multiple, different sites of the solid support, wherein said oligonucleotide probes have an average size of about 20 to about 100 nucleotides, preferably about 40 to about 85 nucleotides, more preferred about 45 to about 75 nucleotides, in particular about 55 to about 65 nucleotide residues or about 60 nucleotide residues,
ii) a nucleic acid sample comprising target nucleic acid molecules,
(b) amplifying said oligonucleotide probes wherein the amplification products comprise a binding moiety and wherein said amplification products are maintained in solution,
(c1) fragmenting and denaturing the target nucleic acid molecules of said genome to yield fragmented single stranded target sequences having an average size of about 100 to about 1000 nucleotide residues, preferably about 250 to about 800 nucleotide residues and most preferably about 400 to about 600 nucleotide residues, in particular about 500 nucleotide residues, and
(c2) hybridizing the target nucleic acid molecules to said amplification products in solution under specific hybridizing conditions,
(d) separating the hybridization complexes of target nucleic acid molecules and amplification products from non-specifically hybridized nucleic acids by said binding moiety,
(e) separating the target nucleic acid molecules from the complex,
(f) determining the nucleic acid sequence of the separated target nucleic acid molecules by means of performing sequencing by synthesis reactions,
(g) comparing the determined sequence to sequences in a database of the reference genome,
(h) identifying sequences in the determined sequence which do not uniquely map back to the reference genome for the identification of a chimeric sequence, wherein the chimeric sequence maps to different regions in the genome, and
(i) detecting at least one balanced chromosomal aberration.

9. The method according to claim 8, wherein
i) the multiple, different oligonucleotide probes each contain a chemical group or linker being able to bind to a solid support;
ii) said fragmented target nucleic acid molecules further comprise adaptor molecules at one or both ends;
and/or
iii) said oligonucleotide probes further comprise primer binding sequences at one or both ends of said probes, especially wherein said primer binding sequences when present at both ends of the probes are the same or are different.

10. The method according to at least one of the claims 8-9, wherein
i) said amplifying comprises exponential polymerase chain reaction, in particular exponential polymerase chain reaction and further asymmetric polymerase chain reaction;
ii) binding moiety is a biotin binding moiety;
iii) said separating comprises binding said biotin binding moiety to a streptavidin coated substrate, in particular to a straptavidin coated paramagnetic particle;
and/or
iv) said method further comprises washing said hybridization complexes prior to separating the target nucleic acid molecules from the complex, especially wherein said method further comprises the step of amplification the separated target nucleic acid molecule prior to step (f), in particular by emulsion polymerase chain reaction.

11. The method according to at least one of the claims 8-10, wherein said nucleic acid molecules are genomic DNA molecules, in particular containing the whole genome or at least one chromosome of an organism, or at least one genomic nucleic acid molecule with a size of at least about 200 kb, at least about 500 kb, at least about 1 Mb, at least about 2 Mb or at least about 5 Mb, especially a size between about 100 kb and about 5 Mb, between about 200 kb and about 5 Mb, between about 500 kb and about 5 Mb, between about 1 Mb and about 2 Mb or between 2 Mb and about 5 Mb.

12. The method according to at least one of the claims 8-11, wherein the oligonucleotide probes containing exons, introns and/or regulatory sequences from at least a part of a genome of an organism have a size of at least 1 Mb or at least one of the sizes as specified in claim 8, or wherein the oligonucleotide probes contain exons, introns and/or regulatory sequences from at least one chromosome of an organism, preferably from at least about 90%, in particular at least about 95%, especially at least about 98% of the genome of an organism, in particular a human genome.

13. The method according to at least one of the claims 8-12, wherein
i) probes with highly repetitive sequences are excluded;
ii) the database of the reference genome contains the whole genome, at least one chromosome of an organism, or at least about 90%, in particular at least about 95%, especially at least about 98% of the genome or of at least one chromosome of an organism, in particular a human genome or chromosome;
iii) any primer or adaptor sequence is removed *in silico* prior to step (f);
and/or
iv) said solid support is either a nucleic acid microarray or a population of beads.

14. The method according to at least one of the claims 1-13, wherein the balanced chromosomal aberration is a translocation or inversion.

15. The method according to at least one of the claims 1-14, further including the detection of at least one further mutation, in particular at least one further deletion, especially a deletion in the breakpoint area of the translocation or inversion, at least one further insertion and/or at least one further substitution in the genome, e.g. at least one single nucleotide polymorphism (SNP).

## Patentansprüche

1. Verfahren zum Detektieren ausgeglichener chromosomaler Aberrationen in einem Genom, wobei das Verfahren die Schritte umfasst:
(a) Aussetzen von fragmentierten, denaturierten Nukleinsäuremolekülen des Genoms an multiple, unterschiedliche Oligonukleotidsonden, die Exons, Introns und/oder regulatorische Sequenzen von wenigstens einem Teil eines Genoms eines Organismus mit einer Größe von wenigstens 50 kb oder von wenigstens einem Gen oder wenigstens einem Chromosom eines Organismus enthalten, wobei die Oligonukleotidsonden auf multiplen, unterschiedlichen Stellen eines festen Trägers unter hybridisierenden Bedingungen lokalisiert sind, um Nukleinsäuremoleküle, die spezifisch an die Sonden hybridisieren, zu fangen,
wobei die fragmentieren, denaturierten Nukleinsäuremoleküle eine durchschnittliche Größe von etwa 100 bis etwa 1000 Nukleotidresten, bevorzugt etwa 250 bis etwa 800 Nukleotidresten und am bevorzugtesten etwa 400 bis etwa 600 Nukleotidresten, insbesondere etwa 500 Nukleotidresten haben,
wobei die Oligonukleotidsonden eine durchschnittliche Größe von etwa 20 bis etwa 100 Nukleotiden, bevorzugt etwa 40 bis etwa 85 Nukleotiden, stärker bevorzugt etwa 45 bis etwa 75 Nukleotiden, insbesondere etwa 55 bis etwa 65 Nukleotidresten oder etwa 60 Nukleotidresten haben,
(b) Abtrennen von ungebundener und unspezifisch hybridisierten Nukleinsäuren von den gefangenen Molekülen,
(c) Eluieren der gefangenen Moleküle von dem festen Träger,
(d) optionales Wiederholen der Schritte (a) bis (c) für wenigstens einen weiteren Zyklus mit den eluierten gefangenen Molekülen,
(e) Bestimmen der Nukleinsäuresequenz der gefangenen Moleküle mittels Durchführen von Sequenzieren durch Synthesereaktionen,
(f) Vergleichen der bestimmten Sequenz mit Sequenzen in einer Datenbank des Referenzgenoms,
(g) Identifizieren von Sequenzen in der bestimmten Sequenz, welche nicht eindeutig auf das Referenzgenom zurückkartieren, für die Identifikation einer chimären Sequenz, wobei die chimäre Sequenz auf unterschiedliche Regionen in dem Genom kartiert, und
(h) Detektieren wenigstens einer ausgeglichenen chromosomalen Aberration.

2. Verfahren gemäß Anspruch 1, wobei die multiplen, unterschiedlichen Oligonukleotidsonden jeweils eine chemische Gruppe oder einen Linker enthalten, die/der geeignet ist, an einen festen Träger zu binden.

3. Verfahren gemäß Anspruch 1 oder 2, ferner umfassend den Schritt des Ligierens eines Adaptormoleküls an ein oder beide, bevorzugt beide, Enden der Nukleinsäuremoleküle vor oder nach Schritt (a);
besonders ferner umfassend den Schritt der Amplifikation der Nukleinsäuremoleküle mit wenigstens einem Primer, wobei der Primer eine Sequenz, welche spezifisch mit der Sequenz des Adaptormoleküls/der Adaptormoleküle hybridisiert, umfasst, wobei der Schritt der Amplifikation nach Schritt (c) oder nach Schritt (d) ausgeführt wird.

4. Verfahren gemäß wenigstens einem der Ansprüche 1 bis 3, ferner umfassend den Schritt des Reinigens der Nukleinsäuremoleküle vor Schritt (e);
besonders ferner umfassend den Schritt der Amplifikation des gereinigten Nukleinsäuremoleküls vor Schritt (e), insbesondere durch Emulsions-Polymerasekettenreaktion.

5. Verfahren gemäß wenigstens einem der Ansprüche 1-4, wobei die Nukleinsäuremoleküle genomische DNA-Moleküle sind, die insbesondere das gesamte Genom oder wenigstens ein Chromosom eines Organismus oder wenigstens ein genomisches Nukleinsäuremolekül mit einer Größe von wenigstens etwa 50 kb, wenigstens etwa 200 kb, wenigstens etwa 500 kb, wenigstens etwa 1 Mb, wenigstens etwa 2 Mb oder wenigstens etwa 5 Mb, besonders einer Größe zwischen etwa 100 kb und etwa 5 Mb, zwischen etwa 200 kb und etwa 5 Mb, zwischen etwa 500 kb und etwa 5 Mb, zwischen etwa 1 Mb und etwa 2 Mb oder zwischen etwa 2 Mb und etwa 5 Mb enthalten.

6. Verfahren gemäß wenigstens einem der Ansprüche 1-5, wobei die Oligonukleotidsonden, die Exons, Introns und/oder regulatorische Sequenzen von wenigstens einem Teil des Genoms eines Organismus enthalten, eine Größe von wenigstens 100 kb, bevorzugt wenigstens 1 Mb oder wenigstens eine der Größen wie in Anspruch 8 spezifiziert haben, oder wobei die Oligonukleotidsonden Exons, Introns und/oder regulatorische Sequenzen von wenigstens etwa 90 %, insbesondere wenigstens etwa 95 %, besonders wenigstens 98 % des Gens oder Genoms eines Organismus, insbesondere eines humanen Gens oder Genoms, enthalten.

7. Verfahren gemäß wenigstens einem der Ansprüche 1-6, wobei
i) Sonden mit hochrepetitiven Sequenzen ausgeschlossen sind;
ii) die Datenbank des Referenzgenoms das gesamte Genom oder wenigstens ein Chromosom eines Organismus oder wenigstens etwa 90 %, insbesondere wenigstens etwa 95 %, besonders wenigstens etwa 98 % des Genoms oder wenigstens eines Chromosoms eines Organismus, insbesondere eines humanen Genoms oder Chromosoms, enthält;
iii) jede Primer- oder Adaptorsequenz vor Schritt (f) *in silico* entfernt wird; und/oder
iv) der feste Träger entweder ein Nukleinsäuremicroarray oder ein Bestand an Kügelchen ist.

8. Verfahren zum Detektieren ausgeglichener chromosomaler Aberrationen in einem Genom, wobei das Verfahren die Schritte umfasst:
(a) Bereitstellen:
i) eines festen Trägers, der multiple, unterschiedliche Oligonukleotidsonden, die Exons, Introns und/oder regulatorische Sequenzen von wenigstens einem Teil eines Genoms eines Organismus mit einer Größe von wenigstens 100 kb von wenigstens einem Chromosom eines Organismus enthalten, wobei die Oligonukleotidsonden auf multiplen, unterschiedlichen Stellen des festen Trägers lokalisiert sind, wobei die Oligonukleotidsonden eine durchschnittliche Größe von etwa 20 bis etwa 100 Nukleotiden, bevorzugt etwa 40 bis etwa 85 Nukleotiden, stärker bevorzugt etwa 45 bis etwa 75 Nukleotiden, insbesondere etwa 55 bis etwa 65 Nukleotidresten oder etwa 60 Nukleotidresten haben,
ii) einer Nukleinsäureprobe, die Zielnukleinsäuremoleküle umfasst,
(b) Amplifizieren der Oligonukleotidsonden, wobei die Amplifikationsprodukte einen Bindungsrest enthalten und wobei die Amplifikationsprodukte in Lösung gehalten werden,
(c1) Fragmentieren und Denaturieren der Nukleinsäuremoleküle des Genoms, um fragmentierte einzelsträngige Zielsequenzen mit einer durchschnittlichen Größe von etwa 100 bis etwa 1000 Nukleotidresten, bevorzugt etwa 250 bis etwa 800 Nukleotidresten und am bevorzugtesten etwa 400 bis etwa 600 Nukleotidresten, insbesondere etwa 500 Nukleotidresten, zu ergeben, und
(c2) Hybridisieren der Zielnukleinsäuremoleküle an die
Amplifikationsprodukte in Lösung unter spezifischen Hybridisierungsbedingungen,
(d) Abtrennen der Hybridisierungskomplexe der Zielnukleinsäuremoleküle und Amplifikationsprodukte von unspezifisch hybridisierten Nukleinsäuren durch den Bindungsrest,
(e) Abtrennen der Zielnukleinsäuremoleküle von dem Komplex,
(f) Bestimmen der Nukleinsäuresequenz der abgetrennten Zielnukleinsäuremoleküle mittels Durchführen von Sequenzieren durch Synthesereaktionen,
(g) Vergleichen der bestimmten Sequenz mit Sequenzen in einer Datenbank des Referenzgenoms,
(h) Identifizieren von Sequenzen in der bestimmten Sequenz, welche nicht eindeutig auf das Referenzgenom zurückkartieren, für die Identifikation einer chimären Sequenz, wobei die chimäre Sequenz auf unterschiedliche Regionen in dem Genom kartiert, und
(i) Detektieren wenigstens einer ausgeglichenen chromosomalen Aberration.

9. Verfahren gemäß Anspruch 8, wobei
i) die multiplen, unterschiedlichen Oligonukleotidsonden jeweils eine chemische Gruppe oder einen Linker enthalten, die/der geeignet ist, an einen festen Träger zu binden;
ii) die fragmentierten Zielnukleinsäuremoleküle ferner Adaptormoleküle an einem oder beiden Enden enthalten;
und/oder
iii) die Oligonukleotidsonden ferner Primer-bindende Sequenzen an einem oder beiden Enden der Sonden umfassen, besonders wobei die Primerbindenden Sequenzen die gleichen sind oder unterschiedliche sind, wenn sie an beiden Enden der Sonden vorhanden sind.

10. Verfahren gemäß wenigstens einem der Ansprüche 8-9, wobei
i) das Amplifizieren exponentielle Polymerasekettenreaktion, insbesondere exponentielle Polymerasekettenreaktion und ferner asymmetrische Polymerasekettenreaktion, umfasst;
ii) Bindungsrest ein Biotinbindungsrest ist;
iii) das Abtrennen das Binden des Biotinbindungsrests an ein Streptavidinbeschichtetes Substrat, insbesondere an einen Streptavidin-beschichteten paramagnetischen Partikel, umfasst;
und/oder
iv) das Verfahren ferner das Waschen der Hybridisierungskomplexe vor dem Abtrennen der Zielnukleinsäuremoleküle von dem Komplex umfasst, besonders wobei das Verfahren ferner den Schritt der Amplifikation des abgetrennten Zielnukleinsäuremoleküls vor Schritt (f), insbesondere durch Emulsions-Polymerasekettenreaktion, umfasst.

11. Verfahren gemäß wenigstens einem der Ansprüche 8-10, wobei die Nukleinsäuremoleküle genomische DNA-Moleküle sind, die insbesondere das gesamte Genom oder wenigstens ein Chromosom eines Organismus oder wenigstens ein genomisches Nukleinsäuremolekül mit einer Größe von wenigstens etwa 200 kb, wenigstens etwa 500 kb, wenigstens etwa 1 Mb, wenigstens etwa 2 Mb oder wenigstens etwa 5 Mb, besonders einer Größe zwischen etwa 100 kb und etwa 5 Mb, zwischen etwa 200 kb und etwa 5 Mb, zwischen etwa 500 kb und etwa 5 Mb, zwischen etwa 1 Mb und etwa 2 Mb oder zwischen 2 Mb und etwa 5 Mb enthalten.

12. Verfahren gemäß wenigstens einem der Ansprüche 8-11, wobei die Oligonukleotidsonden, die Exons, Introns und/oder regulatorische Sequenzen von wenigstens einem Teil des Genoms eines Organismus enthalten, eine Größe von wenigstens 1 Mb oder wenigstens eine der Größen wie in Anspruch 8 spezifiziert haben, oder wobei die Oligonukleotidsonden Exons, Introns und/oder regulatorische Sequenzen von wenigstens einem Chromosom eines Organismus, bevorzugt von wenigstens etwa 90 %, insbesondere wenigstens etwa 95 %, besonders wenigstens etwa 98 % des Genoms eines Organismus, insbesondere eines humanen Genoms, enthalten.

13. Verfahren gemäß wenigstens einem der Ansprüche 8-12, wobei
i) Sonden mit hochrepetitiven Sequenzen ausgeschlossen sind;
ii) die Datenbank des Referenzgenoms das gesamte Genom, wenigstens ein Chromosom eines Organismus oder wenigstens etwa 90 %, insbesondere wenigstens etwa 95 %, besonders wenigstens etwa 98 % des Genoms oder wenigstens eines Chromosoms eines Organismus, insbesondere eines humanen Genoms oder Chromosoms, enthält;
iii) jede Primer- oder Adaptorsequenz vor Schritt (f) *in silico* entfernt wird; und/oder
iv) der feste Träger entweder ein Nukleinsäuremicroarray oder ein Bestand an Kügelchen ist.

14. Verfahren gemäß wenigstens einem der Ansprüche 1-13, wobei die ausgeglichene chromosomale Aberration eine Translokation oder Inversion ist.

15. Verfahren gemäß wenigstens einem der Ansprüche 1-14, ferner einschließend die Detektion von mindestens einer weiteren Mutation, insbesondere einer weiteren Deletion, besonders einer Deletion im Stoppstellenbereich der Translokation oder Inversion, wenigstens eine weitere Insertion und/oder wenigstens eine weitere Substitution in das/dem Genom, z.B. bei wenigstens einem Einzelnukleotidpolymorphismus ("single nucleotide polymorphism")(SNP).

## Revendications

1. Procédé pour détecter des anomalies chromosomiques équilibrées dans un génome, le procédé comprenant les étapes dans lesquelles :
(a) on expose des molécules fragmentées d'acide nucléique dénaturé dudit génome à plusieurs sondes oligonucléotidiques différentes contenant des exons, des introns et/ou des séquences régulatrices issus d'au moins une partie du génome d'un organisme possédant une taille d'au moins 50 kb ou d'au moins un gène ou d'au moins un chromosome d'un organisme, les sondes oligonucléotidiques étant disposées sur plusieurs sites différents d'un support solide dans des conditions d'hybridation afin de capturer les molécules d'acide nucléique qui s'hybrident de manière spécifique auxdites sondes ;
dans lequel lesdites molécules fragmentées d'acide nucléique dénaturé possèdent une taille moyenne d'environ 100 à environ 1000 résidus nucléotidiques, de préférence d'environ 250 à environ 800 résidus nucléotidiques et de manière de loin préférée d'environ 400 à environ 600 résidus nucléotidiques, en particulier environ 500 résidus nucléotidiques ;
dans lequel lesdites sondes oligonucléotidiques possèdent une taille moyenne d'environ 20 à environ 100 nucléotides, de préférence d'environ 40 à environ 85 nucléotides, de manière plus préférée d'environ 45 à environ 75 nucléotides, en particulier d'environ 55 à environ 65 résidus nucléotidiques, ou environ 60 résidus nucléotidiques ;
(b) on sépare, des molécules capturées, les acides nucléiques non liés et non hybridés de manière spécifique ;
(c) on élue les molécules capturées à partir du support solide ;
(d) on répète de manière facultative les étapes (a) à (c) pendant au moins un cycle supplémentaire avec les molécules capturées éluées ;
(e) on détermine la séquence d'acides nucléiques des molécules capturées en procédant à un séquençage via des réactions de synthèse ;
(f) on compare la séquence déterminée à des séquences dans une base de données du génome de référence ;
(g) on identifie des séquences, dans la séquence déterminée, qui ne manifestent pas une correspondance univoque par rapport au génome de référence pour l'identification d'une séquence chimère, la séquence chimère correspondant à différentes régions dans le génome ; et
(h) on détecte au moins une anomalie chromosomique équilibrée.

2. Procédé selon la revendication 1, dans lequel lesdites plusieurs sondes oligonucléotidiques différentes contiennent chacune un groupe chimique ou un lieur capable de se lier à un support solide.

3. Procédé selon la revendication 1 ou 2, comprenant l'étape dans laquelle on procède à une ligature de molécules adaptatrices à une extrémité des molécules d'acides nucléiques ou aux deux, de préférence auxdites deux extrémités, avant ou après l'étape (a) ;
en particulier comprenant en outre l'étape dans laquelle on procède à une amplification desdites molécules d'acides nucléiques avec au moins une amorce, ladite amorce comprenant une séquence qui s'hybride de manière spécifique à la séquence de ladite/desdites molécules adaptatrices, l'étape d'amplification étant mise en oeuvre après l'étape (c) ou après l'étape (d).

4. Procédé selon au moins une des revendications 1 à 3, comprenant en outre l'étape dans laquelle on purifie lesdites molécules d'acides nucléiques avant l'étape (e) ;
en particulier comprenant en outre l'étape dans laquelle on amplifie la molécule purifiée d'acides nucléiques avant l'étape (e), en particulier via une réaction en chaîne par polymérase en émulsion.

5. Procédé selon au moins une des revendications 1 à 4, dans lequel lesdites molécules d'acides nucléiques sont des molécules d'ADN génomique, en particulier contenant le génome entier ou au moins un chromosome d'un organisme, ou au moins une molécule d'acides nucléiques génomiques possédant une taille d'au moins environ 50 kb, au moins d'environ 200 kb, au moins d'environ 500 kb, au moins d'environ 1 Mb, au moins d'environ 2 Mb ou au moins d'environ 5 Mb, en particulier une taille entre environ 100 kb et environ 5 Mb, entre environ 200 kb et environ 5 Mb, entre environ 500 kb et environ 5 Mb, entre environ 1 Mb et environ 2 Mb et entre environ 2 Mb et environ 5 Mb.

6. Procédé selon au moins une des revendications 1 à 5, dans lequel les sondes oligonucléotidiques contenant des exons, des introns et/ou des séquences régulatrices issus d'au moins une partie du génome d'un organisme possèdent une taille d'au moins 100 kb, de préférence d'au moins 1 Mb ou au moins une des tailles telles que spécifiées à la revendication 8, ou dans lequel les sondes oligonucléotidiques contiennent des exons, des introns et/ou des séquences régulatrices issues d'au moins environ 90 %, en particulier d'au moins environ 95 %, de manière spécifique d'au moins environ 98 % du gène ou du génome d'un organisme, en particulier d'un gène ou d'un génome humain.

7. Procédé selon au moins une des revendications 1 à 6, dans lequel
i) des sondes comprenant des séquences fortement répétitives sont exclues ;
ii) la base de données du génome de référence contient le génome entier ou au moins un chromosome d'un organisme, ou au moins environ 90 %, en particulier au moins environ 95 %, de manière spécifique au moins environ 98 % du génome ou d'au moins un chromosome d'un organisme, en particulier un génome ou un chromosome humain ;
iii) n'importe quelle amorce ou séquence adaptatrice est éliminée *in silico* avant l'étape (f) ;
et/ou
iv) ledit support solide représente soit une micropuce d'acides nucléiques, soit une population de billes.

8. Procédé pour détecter des anomalies chromosomiques équilibrées dans un génome, le procédé comprenant les étapes dans lesquelles :
(a) on procure :
(i) un support solide comprenant plusieurs sondes oligonucléotidiques différentes contenant des exons, des introns et/ou des séquences régulatrices issus d'au moins une partie du génome d'un organisme possédant une taille d'au moins 100 kb, ou d'au moins un chromosome d'un organisme, les sondes oligonucléotidiques étant situées sur plusieurs sites différents du support solide, lesdites sondes oligonucléotidiques possédant une taille moyenne d'environ 20 à environ 100 nucléotides, de préférence d'environ 40 à environ 85 nucléotides, de manière plus préférée d'environ 45 à environ 75 nucléotides, en particulier d'environ 55 à environ 65 résidus nucléotidiques, ou environ 60 résidus nucléotidiques ;
(ii) un échantillon d'acides nucléiques comprenant des molécules cibles d'acides nucléiques ;
(b) on amplifie lesdites sondes oligonucléotidiques, les produits de l'amplification comprenant une fraction de liaison et lesdits produits d'amplification étant maintenus en solution ;
(c1) on fragmente et on dénature les molécules cibles d'acides nucléiques dudit génome pour obtenir des séquences cibles fragmentées simple brin possédant une taille moyenne d'environ 100 à environ 1000 résidus nucléotidiques, de préférence d'environ 250 à environ 800 résidus nucléotidiques et de manière de loin préférée d'environ 400 à environ 600 résidus nucléotidiques, en particulier environ 500 résidus nucléotidiques ; et
(c2) on hybride les molécules cibles d'acides nucléiques auxdits produits d'amplification en solution dans des conditions d'hybridation spécifiques ;
(d) on sépare les complexes d'hybridation des molécules cibles d'acides nucléiques et des produits d'amplification par rapport aux acides nucléiques non hybridés de manière spécifique via ladite fraction de liaison ;
(e) on sépare les molécules cibles d'acides nucléiques par rapport au complexe ;
(f) on détermine la séquence d'acides nucléiques des molécules cibles séparées d'acides nucléiques en procédant à un séquençage via des réactions de synthèse ;
(g) on compare la séquence déterminée à des séquences dans une base de données du génome de référence ;
(h) on identifie des séquences, dans la séquence déterminée, qui ne manifestent pas une correspondance univoque par rapport au génome de référence pour l'identification d'une séquence chimère, la séquence chimère correspondant à différentes régions dans le génome ; et
(i) on détecte au moins une anomalie chromosomique équilibrée.

9. Procédé selon la revendication 8, dans lequel
(i) lesdites plusieurs sondes oligonucléotidiques différentes contiennent chacune un groupe chimique ou un lieur capable de se lier à un support solide ;
(ii) lesdites molécules cibles fragmentées d'acides nucléiques comprennent en outre des molécules adaptatrices à une extrémité ou aux deux extrémités ;
et/ou
iii) lesdites sondes oligonucléotidiques comprennent en outre des séquences de liaison faisant office d'amorce à une extrémité lesdites sondes ou auxdites deux extrémités, en particulier dans lequel lesdites séquences de liaison faisant office d'amorce, lorsqu'elles sont présentes aux deux extrémités des sondes, sont identiques ou sont différentes.

10. Procédé selon au moins une des revendications 8 à 9, dans lequel
i) ladite amplification comprend une réaction exponentielle en chaîne par polymérase, en particulier une réaction exponentielle en chaîne par polymérase et en outre une réaction asymétrique en chaîne par polymérase ;
ii) la fraction de liaison est une fraction de liaison à base de biotine ;
iii) ladite séparation comprend la liaison de ladite fraction de liaison à base de biotine à un substrat enduit de streptavidine, en particulier à une particule paramagnétique enduite de streptavidine ;
et/ou
iv) ledit procédé comprend en outre le lavage desdits complexes d'hybridation avant la séparation des molécules cible d'acides nucléiques par rapport au complexe, en particulier dans lequel ledit procédé comprend en outre l'étape dans laquelle on amplifie les molécules cibles séparées d'acides nucléiques avant l'étape (f), en particulier via une réaction en chaîne par polymérase en émulsion.

11. Procédé selon au moins une des revendications 8 à 10, dans lequel lesdites molécules d'acides nucléiques sont des molécules d'ADN génomique en particulier contenant le génome entier ou au moins un chromosome d'un organisme, ou au moins une molécule d'acides nucléiques génomiques possédant une taille d'au moins environ 200 kb, au moins d'environ 500 kb, au moins d'environ 1 Mb, au moins d'environ 2 Mb ou au moins d'environ 5 Mb, en particulier une taille entre environ 100 kb et environ 5 Mb, entre environ 200 kb et environ 5 Mb, entre environ 500 kb et environ 5 Mb, entre environ 1 Mb et environ 2 Mb et entre environ 2 Mb et environ 5 Mb.

12. Procédé selon au moins une des revendications 8 à 11, dans lequel les sondes oligonucléotidiques contenant des exons, des introns et/ou des séquences régulatrices issus d'au moins une partie du génome d'un organisme possèdent une taille d'au moins 1Mb, ou au moins une des tailles telles que spécifiées à la revendication 8, ou dans lequel les sondes oligonucléotidiques contiennent des exons, des introns et/ou des séquences régulatrices issues d'au moins un chromosome d'un organisme, de préférence d'au moins environ 90 %, en particulier d'au moins environ 95 %, de manière spécifique d'au moins environ 98 % du génome d'un organisme, en particulier d'un génome humain.

13. Procédé selon au moins une des revendications 8 à 12, dans lequel
i) des sondes comprenant des séquences fortement répétitives sont exclues ;
ii) la base de données du génome de référence contient le génome entier ou au moins un chromosome d'un organisme, ou au moins environ 90 %, en particulier au moins environ 95 %, de manière spécifique au moins environ 98 % du génome ou d'au moins un chromosome d'un organisme, en particulier un génome ou un chromosome humain ;
iii) n'importe quelle amorce ou séquence adaptatrice est éliminée *in silico* avant l'étape (f) ;
et/ou
iv) ledit support solide représente soit une micropuce d'acides nucléiques, soit une population de billes.

14. Procédé selon au moins une des revendications 1 à 13, dans lequel l'anomalie chromosomique équilibrée est une translocation ou une inversion.

15. Procédé selon au moins une des revendications 1 à 14, englobant en outre la détection d'au moins une mutation supplémentaire, en particulier d'au moins une délétion supplémentaire, en particulier une délétion dans la zone du point d'interruption de la translocation ou de l'inversion, au moins une insertion supplémentaire et/ou au moins une substitution supplémentaire dans le génome, par exemple au moins un polymorphisme nucléotidique (SNP).
